(19) Europäisches Patentamt
European Patent Office
Office européen des brevets

(11) EP 3 224 379 B1

(12) **FASCICULE DE BREVET EUROPEEN**

(45) Date de publication et mention de la délivrance du brevet:
**01.04.2020 Bulletin 2020/14**

(21) Numéro de dépôt: **15808739.5**

(22) Date de dépôt: **26.11.2015**

(51) Int Cl.:
***C12Q 1/6886*** *(2018.01)*

(86) Numéro de dépôt international:
**PCT/FR2015/053218**

(87) Numéro de publication internationale:
**WO 2016/083742 (02.06.2016 Gazette 2016/22)**

(54) **MÉTHODE DE PRONOSTIC D'HÉMOPATHIES**

VERFAHREN ZUR PROGNOSE VON HÄMOPATHIEN

HEMOPATHY PROGNOSIS METHOD

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **27.11.2014 FR 1461553**

(43) Date de publication de la demande:
**04.10.2017 Bulletin 2017/40**

(73) Titulaires:
- **CENTRE NATIONAL DE LA RECHERCHE SCIENTIFIQUE (CNRS)**
  **75794 Paris cedex 16 (FR)**
- **Université François-Rabelais**
  **37020 Tours Cedex 1 (FR)**

(72) Inventeur: **HERAULT, Olivier**
**37250 La Tremblaye (FR)**

(74) Mandataire: **Monni, Richard**
**Cabinet LLR**
**11 boulevard de Sébastopol**
**75001 Paris (FR)**

(56) Documents cités:
**WO-A1-2012/049329** **WO-A1-2012/085188**
**US-A1- 2007 154 931**

- **O. HERAULT ET AL: "A role for GPx3 in activity of normal and leukemia stem cells", BLOOD, vol. 117, no. 2, 16 avril 2012 (2012-04-16), pages 895-901, XP055203622, ISSN: 0006-4971, DOI: 10.1182/blood-2010-07-293332**
- **Anonymous: "RT 2 Profiler (TM) PCR Array: Human Oxidative Stress and Antioxidant Defense", , 22 septembre 2007 (2007-09-22), pages 1-4, XP055203631, Extrait de l'Internet: URL:http://www.biomol.de/details/SA/DS/PAHS-065A.pdf [extrait le 2015-07-21]**
- **RIZWAN AHMAD ET AL: "OXIDATIVE STRESS AND ANTIOXIDANT STATUS IN PATIENTS WITH CHRONIC MYELOID LEUKEMIA", INDIAN JOURNAL OF CLINICAL BIOCHEMISTRY, vol. 23, no. 4, 1 janvier 2008 (2008-01-01), pages 328-333, XP055203451,**
- **W. YAN ET AL: "GPX2, a Direct Target of p63, Inhibits Oxidative Stress-induced Apoptosis in a p53-dependent Manner", JOURNAL OF BIOLOGICAL CHEMISTRY, vol. 281, no. 12, 24 mars 2006 (2006-03-24), pages 7856-7862, XP055203509, ISSN: 0021-9258, DOI: 10.1074/jbc.M512655200**
- **CHIARA GORRINI ET AL: "Modulation of oxidative stress as an anticancer strategy", NATURE REVIEWS DRUG DISCOVERY, vol. 12, no. 12, 29 novembre 2013 (2013-11-29), pages 931-947, XP055203511, ISSN: 1474-1776, DOI: 10.1038/nrd4002**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 224 379 B1

## Description

La présente invention concerne une méthode de pronostic d'hémopathies, plus particulièrement le pronostic personnalisé, ou théranostic, des hémopathies.

La leucémie myéloïde chronique fait partie des maladies du sang regroupées sous le nom de « syndromes myéloprolifératifs ». Elle se caractérise par une production excessive et persistante au sein de la moelle osseuse des globules blancs anormaux (des polynucléaires neutrophiles pathologiques). Sans traitement adapté, elle évolue en leucémie aiguë, caractérisée par l'accumulation de cellules immatures dans la moelle osseuse.

La leucémie myéloïde chronique (LMC) est une maladie relativement rare, puisque l'on compte environ 600 nouveaux cas par an en France. Elle est un peu plus fréquente chez l'homme que chez la femme. Sa fréquence augmente avec l'âge. Au moment du diagnostic, l'âge médian des patients est de 53 ans.

La maladie est liée à l'apparition d'une anomalie liée à une translocation entre les chromosomes 9 et 22 dans des cellules souches de la moelle osseuse, provoquant l'apparition d'un petit chromosome anormal, le chromosome Philadelphie (du nom de la ville des Etats-Unis où a eu lieu le congrès au cours duquel a été présenté pour la première fois l'anomalie chromosomique - en 1960). Cette anomalie induit l'assemblage par erreur d'un gène du chromosome 9, dénommée ABL, avec un gène du chromosome 22, nommé BCR. Cela produit le gène dit BCR-ABL qui est présent uniquement dans les cellules pathologiques. Ce gène produit une quantité anormalement élevée d'une enzyme, la tyrosine kinase Abelson (ABL) responsable de la production accrue des globules blancs porteurs du chromosome de Philadelphie.

La leucémie myéloïde chronique évolue en trois phases :

- La phase chronique.
  C'est à ce stade que la maladie est diagnostiquée chez la plupart des malades. Pendant cette phase, la leucémie évolue lentement et il n'y a pas ou peu de symptômes. Il y a encore peu de globules blancs immatures (leucoblastes) dans la moelle osseuse et dans le sang. Cette phase dure en moyenne de trois à quatre ans en l'absence de traitement.
- La phase d'accélération.
  Elle correspond à une augmentation de la proportion de leucoblastes dans le sang et dans la moelle, ainsi qu'à une élévation de la charge BCR-ABL et l'apparition de nouvelles anomalies chromosomiques. Des symptômes, non spécifiques, sont plus fréquents, tels que fatigue, perte d'appétit, fièvre sans raison apparente. Si un traitement n'est pas mis en œuvre, la maladie évolue en quelques mois vers la phase aiguë, dite de transformation.
- La phase de transformation.

De chronique, la leucémie devient alors aiguë. La moelle osseuse est envahie par des leucoblastes et ne peut plus fonctionner correctement. Le pronostic de cette leucémie aiguë secondaire est très mauvais à court terme.

La prise en charge thérapeutique de la leucémie myéloïde chronique repose sur l'administration de médicaments appelés inhibiteurs de la tyrosine kinase.

L'objectif du traitement est de prévenir la transformation aiguë et sa finalité est l'éradication des cellules exprimant BCR-ABL et d'éviter la transformation en leucémie aiguë.

Au cours de la phase chronique, le traitement de première intention est constitué par l'imatinib mésylate (Glivec®). D'autres inhibiteurs de tyrosine kinase, dits de seconde génération ont été développés après l'imatinib mesylate: le nilotinib et le dasatinib. Ils sont potentiellement générateurs d'effets secondaires graves. Le nilotinib à une AMM en première ligne.

L'objectif du traitement est l'obtention d'une réponse moléculaire majeure le plus rapidement possible pour éviter une sélection clonale. Cette réponse moléculaire suit la rémission cytologique et cytogénétique.

Il est donc nécessaire de fournir au patient un traitement approprié le plus rapidement possible au patient, permettant l'obtention rapide d'une réponse moléculaire majeure sans induire d'effet secondaire grave. Il existe plusieurs inhibiteurs de tyrosine kinase et un choix thérapeutique inadapté dans la première année aura par ailleurs un coût très élevé pour le système de santé.

Afin d'optimiser les choix thérapeutiques, des méthodes de pronostic visant notamment à évaluer le risque de résistance à un traitement par les inhibiteurs de tyrosine kinases (ITK) ont été développées. On peut citer par exemple la demande internationale WO 2012/049329 qui vise à prédire la réponse d'un patient à un traitement ITK en mesurant l'activité kinase sur différents substrats.

Toutefois, une telle méthode est difficile à mettre en œuvre simplement et de manière routinière.

Aussi, le besoin de fournir une méthode de pronostic personnalisé de la LMC demeure, puisqu'il n'existe pas de marqueur permettant d'optimiser simplement la décision thérapeutique entre un traitement ITK de première génération et un traitement ITK de seconde génération ou de générations ultérieures.

Un des buts de l'invention est de palier ces inconvénients.

Un autre but de l'invention est de proposer une méthode de pronostic ayant pour objectif de minimiser le risque d'échec thérapeutique.

Encore un autre but de l'invention est de réduire le coût de traitement en proposant en première intention le traitement le plus approprié au patient.

Aussi, l'invention concerne une méthode de pronostic *in vitro* de la réponse à une thérapie d'un individu atteint d'une leucémie myéloïde chronique, à partir d'un échantillon biologique leucémique issu dudit individu, ladite méthode comprenant :

a. une étape de mesure du niveau d'expression des gènes d'au moins un sous-groupe de gènes choisis dans un groupe de gènes,
ledit groupe de gènes étant constitué de 25 gènes, lesdits 25 gènes comprenant ou étant constitués par les séquences d'acides nucléiques SEQ ID NO : 1 à SEQ ID NO : 25,
ledit sous-groupe consistant en 7 gènes, lesdits 7 gènes comprenant ou étant constitués par les séquences d'acides nucléiques SEQ ID NO : 1 à SEQ ID NO : 7,
une valeur du niveau d'expression mesuré étant obtenue pour chacun des gènes dudit sous-groupe,
b. une étape de comparaison de la valeur attribuée à l'étape précédente à chacun desdits gènes dudit sous-groupe à la valeur attribuée à chacun desdits gènes dudit sous-groupe obtenue à partir d'un échantillon biologique sain, afin d'obtenir un ratio pour chacun desdits gènes dudit sous-groupe du niveau d'expression dans l'échantillon biologique leucémique sur le niveau d'expression dans l'échantillon sain, et
c. une étape de détermination d'un score S selon la formule 1 suivante

$$S = \sum ratio\ i - \sum ratio\ j \quad \text{(formule 1)},$$

où ratio i et ratio j représentent respectivement les ratios obtenus pour lesdits gènes dudit sous-groupe comprenant ou étant constitués par les séquences d'acides nucléiques SEQ ID NO : i ou SEQ ID NO : j,
où i et j sont des entiers, i variant de 1 à 5 et j variant de 6 à 7,
de sorte que :

- si S est inférieur à 1, ledit individu aura au moins environ 40% de chances de présenter une rémission moléculaire majeure un an après le début d'une thérapie avec un inhibiteur de tyrosine kinase de première génération, et
- si S est supérieur ou égal à 1, ledit individu aura moins d'environ 40% de chances de présenter une rémission moléculaire majeure un an après le début d'une thérapie avec un inhibiteur de tyrosine kinase de première génération.

En d'autres termes, l'invention concerne une méthode de pronostic *in vitro* de la réponse à une thérapie d'un individu atteint d'une leucémie myéloïde chronique, à partir d'un échantillon biologique leucémique issu dudit individu, ladite méthode comprenant :

a. une étape de mesure de la quantité d'ADN complémentaires correspondant aux gènes d'au moins un sous-groupe de gènes choisis dans un groupe de gènes,
ledit groupe de gènes étant constitué de 25 gènes, lesdits 25 gènes comprenant ou étant constitués par les séquences d'acides nucléiques SEQ ID NO : 1 à SEQ ID NO : 25,
ledit sous-groupe consistant en 7 gènes, lesdits 7 gènes étant identifiés par les molécules d'acides nucléiques comprenant ou consistant en les séquences d'acides nucléiques SEQ ID NO : 1 à SEQ ID NO : 7,
une valeur du niveau d'expression mesuré étant obtenue pour chacun des gènes dudit sous-groupe,
b. une étape de comparaison de la valeur attribuée à l'étape précédente à chacun desdits gènes dudit sous-groupe à la valeur attribuée à chacun desdits gènes dudit sous-groupe obtenue à partir d'un échantillon biologique sain, afin d'obtenir un ratio pour chacun desdits gènes dudit sous-groupe du niveau d'expression dans l'échantillon biologique leucémique sur le niveau d'expression dans l'échantillon sain, et
c. une étape de détermination d'un score S selon la formule 1 suivante

$$S = \sum ratio\ i - \sum ratio\ j \quad \text{(formule 1)},$$

où ratio i et ratio j représentent respectivement les ratios obtenus pour lesdits gènes dudit sous-groupe comprenant ou étant constitués par les séquences d'acides nucléiques SEQ ID NO : i ou SEQ ID NO : j, où i et j sont des entiers, i variant de 1 à 5 et j variant de 6 à 7, de sorte que :

- si S est inférieur à 1, ledit individu aura au moins environ 40% de chances de présenter une rémission moléculaire majeure un an après le début d'une thérapie avec un inhibiteur de tyrosine kinase de première génération, et
- si S est supérieur ou égal à 1, ledit individu aura moins d'environ 40% de chances de présenter une rémission moléculaire majeure un an après le début d'une thérapie avec un inhibiteur de tyrosine kinase de première génération.

L'invention est basée sur la constatation surprenante faite par l'inventeur qu'au moins 7 gènes spécifiques comprenant ou étant constitué des séquences SEQ ID NO : 1 à 7, appartenant à un groupe de 25 gènes comprenant ou étant constitué des séquences SEQ ID NO : 1 à 25 sont suffisants pour déterminer le pronostic, à 1 an, c'est-à-dire un an après le début d'un traitement avec un inhibiteur de tyrosine kinases de première génération, suite au diagnostic de la leucémie myéloïde chronique, de patients atteints de leucémie myéloïde chronique et traités avec un inhibiteur de tyrosine kinase de première génération, i.e. traités avec de l'imatinib mesylate (Glivec®).
Les 25 gènes du groupe susmentionné sont des gènes codant des enzymes participant à la détoxification des cellules dans lesquelles des dérivés réactifs de l'oxygène s'accumulent.
La méthode de l'invention est mise en œuvre de la manière suivante :

- à partir d'un échantillon issus d'un patient, notamment un échantillon de sang, on extrait les acides nucléiques, de préférence les acides ribonucléiques (ARN), selon des méthodes connues de l'homme du métier,
- on mesure la quantité des acides nucléiques issus dudit échantillon et qui comprennent ou sont constitués des séquences SEQ ID NO : 1 à 7, cette mesure permet d'obtenir une valeur du niveau d'expression pour chacun desdits gènes,
- la valeur obtenue pour chacun des gènes précédents est comparée à la valeur obtenue pour les mêmes gènes issus d'un échantillon d'un individu indemne de toute affection hématologique, afin d'obtenir pour chacun desdits gènes des valeurs normalisées, ou ratios,
- lesdites valeurs normalisées sont sommées selon la formule 1 susmentionné, qui peut se résumer par

$$S = \sum ratio\ gène\ SEQ\ ID\ NO{:}\ i - \sum ratio\ ratio\ gène\ SEQ\ ID\ NO{:}\ j$$

  où i varie de 1 à 5 et j varie de 5 à 6, afin d'obtenir le score S,
- on détermine le pronostic selon la valeur obtenue pour le score S obtenu à l'étape précédente.

Dans l'invention, on entend par « un échantillon biologique sain », ou par « un échantillon biologique indemne », un échantillon dont le matériel biologique qu'il contient provient d'un ou plusieurs individus sains, ou au moins dépourvu de toute affection hématologique.
Avantageusement, ledit échantillon sain est de même nature que l'échantillon testé. En d'autres termes, si l'échantillon testé est un échantillon de sang, l'échantillon sain sera également un échantillon de sang d'un autre individu. De la même manière, si l'échantillon est un échantillon de moelle osseuse, l'échantillon sain sera également un échantillon de moelle osseuse.
Il est à noter que si l'échantillon sain (ou indemne) peut correspondre à un ensemble d'échantillons issus d'individus indemnes d'affections hématologiques, l'échantillon du patient est unique, et ne correspond jamais à un mélange d'échantillons issus de patients différents.
Avantageusement, l'échantillon biologique, et donc l'échantillon biologique issu d'un individu sain, est un échantillon de sang total, de leucocytes, de cellules mononucléées circulantes ou de moelle osseuse.
Dans le contexte de l'invention, il est nécessaire de mesurer le niveau d'expression des gènes comprenant ou étant constitués des séquences SEQ ID NO : 1 à SEQ ID NO : 7. Il est toutefois possible, sans que cela n'altère la réponse pronostique obtenue, de mesurer l'expression d'un ou plusieurs autres gènes du groupe de gènes comprenant ou étant constitué par les séquences SEQ ID NO : 8 à 25, qui reflètent l'état d'oxydation de l'échantillon.
Lorsque la méthode de pronostic de l'invention est mise en œuvre, le score S permet de déterminer le pronostic, et notamment les chances de survie, d'un patient au bout d'un an, après traitement en première intention avec un inhibiteur de tyrosine kinase de première génération, notamment de l'imatinib, en particulier de l'imatinib mésylate :

- si le score S est inférieur à 1, le patient qui est traité avec l'imatinib mésylate aura environ au moins 40% de chance de présenter, un an après le début du traitement avec ledit imatinib mésylate, une rémission moléculaire majeure, alors que
- si le score S est supérieur ou égal à 1, le pronostic sera plus sévère et le patient qui est traité avec l'imatinib mésylate aura moins d'environ 40% de chance de présenter, un an après le début du traitement avec ledit imatinib mésylate, une rémission moléculaire majeure.

Par « réponse moléculaire majeure », on entend dans l'invention une disparition ou quasi disparition des cellules exprimant des transcrits BCR-ABL, et avantageusement un ratio BCR-ABL/ABL non recombiné inférieur ou égal à 0,1%. L'objectif du traitement donné au patient étant une rémission complète de la maladie, il est bien entendu préférable que le transcrit BCR-ABL ne soit plus du tout détectable. Toutefois, il est actuellement considéré que le ratio susmentionné BCR-ABL/ABL non recombiné inférieur ou égal à 0,1% est très largement satisfaisant. Ceci correspond aux recommandations européennes de suivi des patients atteints d'une leucémie myéloïde chronique, comme cela a été publié dans Baccarani et al. 2013, Blood, 122(6), 872-884. En résumé, ces préconisations sont :

- si le taux de transcrit BCR-ABL/ABL mesuré par PCR quantitative est inférieur ou égal à 10% après trois mois de traitement, inférieur ou égal à 1% après six mois de traitement et inférieur ou égal à 0,1% après douze mois de traitement avec un inhibiteur de tyrosine kinase, le traitement sera optimal,
- alors que si le taux de transcrit BCR-ABL/ABL mesuré par PCR quantitative est supérieur à 10% après six mois de traitement et supérieur à 1% après douze mois de traitement avec un inhibiteur de tyrosine kinase, il est recommandé de changer le traitement.

Si le ratio BCR-ABL/ABL non recombiné est compris entre 0,1 et 10, on parlera alors de « réponse moléculaire intermédiaire ». Enfin si le ratio BCR-ABL/ABL non recombiné est supérieur à 10, on parlera de « mauvais répondeur » ou de « non répondeur ».

Dans l'invention, les gènes pour lesquels le niveau d'expression est mesuré sont représentés par leur ARN messager, obtenu lors de la transcription desdits gènes, ou de leur ADN complémentaire. Bien évidemment, la transcription de gènes est un processus bien connu de l'état de la technique qu'il ne sera donc pas nécessaire de rappeler.

Certains des gènes dont le niveau d'expression est mesuré dans le cadre de l'invention sont capables d'exprimer plusieurs variants, c'est à dire plusieurs molécules d'ARN messager qui diffèrent dans leur séquence. Ces variant sont généralement obtenu par épissage alternatif, ledit épissage permettant d'ajouter, supprimer ou modifier une ou plusieurs parties du produit d'expression dudit gène. Là encore, il n'est pas besoin d'expliquer le mécanisme d'épissage qui est bien connu de l'état de la technique.

Les gènes centraux du procédé selon l'invention sont les suivants :

- le gène CAT représenté par la séquence SEQ ID NO : 1, codant la catalase,
- le gène SOD1 représente par la séquence SEQ ID NO : 2, codant la Superoxide dismutase [Cu-Zn],
- le gène GPX1, représenté par la séquence SEQ ID NO : 3, et en particulier son variant 1, GPX1(1), codant la Glutathion peroxydase 1,
- le gène GPX4(1-2-3), représenté par la séquence SEQ ID NO : 4, et en particulier ses variants 1 à 3, codant la Glutathion peroxydase 4,
- le gène PDRX1 représenté par la séquence SEQ ID NO : 5, et en particulier ses variants 1 à 3, codant la Péroxyrédoxine,
- le gène SOD2(1-2-3), représenté par la séquence SEQ ID NO : 6, codant pour la Superoxyde dismutase 2, et
- le gène GPX2 représenté par la séquence SEQ ID NO : 7, codant la Glutathion peroxydase 2.

En outre, comme indiqué précédemment, du fait de la présence de variants pour certains gènes, il est possible pour mesurer l'expression :

- du gène GPX4(1-2-3) de mesurer l'expression des acides nucléiques comprenant ou étant constitués des séquences SEQ ID NO : 4 et/ou SEQ ID NO : 8 et/ou SEQ ID NO : 9,
- du gène PRDX1 de mesurer l'expression des acides nucléiques comprenant ou étant constitués des séquences SEQ ID NO : 5 et/ou SEQ ID NO : 10 et/ou SEQ ID NO : 11, et
- du gène SOD2(1-2-3) de mesurer l'expression des acides nucléiques comprenant ou étant constitués des séquences SEQ ID NO : 6 et/ou SEQ ID NO : 12 et/ou SEQ ID NO : 13.

Le tableau ci-dessus récapitule les gènes avantageux selon l'invention.

<table>
<tr><th>Nom du gène</th><th>SEQ ID NO</th><th>Nom du gène</th><th>SEQ ID NO</th></tr>
<tr><td>CAT</td><td>SEQ ID NO : 1</td><td rowspan="3">PRDX1</td><td>SEQ ID NO: 5</td></tr>
<tr><td>SOD1</td><td>SEQ ID NO : 2</td><td>SEQ ID NO: 28</td></tr>
<tr><td>GPX1</td><td>SEQ ID NO : 3</td><td>SEQ ID NO: 29</td></tr>
<tr><td rowspan="3">GPX4(1-2-3)</td><td>SEQ ID NO: 4</td><td rowspan="3">SOD2(1-2-3)</td><td>SEQ ID NO: 6</td></tr>
<tr><td>SEQ ID NO : 26</td><td>SEQ ID NO: 30</td></tr>
<tr><td>SEQ ID NO : 27</td><td>SEQ ID NO: 31</td></tr>
<tr><td colspan="2"></td><td>GPX2</td><td>SEQID NO: 7</td></tr>
</table>

Les séquences indiquées ci-dessus correspondent aux séquences des ADN complémentaires correspondant aux gènes indiqués.

Les outils moléculaires utilisés dans l'invention pour mesurer l'expression des gènes présentant des variants sont tels qu'ils permettent de mesurer le niveau d'expression de l'ensemble des variants d'un même gène simultanément.

Aussi, dans le cadre de l'invention, lorsque l'on mesure le niveau d'expression du gène GPX4(1-2-3) représenté par la séquence SEQ ID NO: 1, on mesure en fait simultanément l'expression des variants dudit gène, c'est-à-dire que l'on mesure simultanément le niveau d'expression des molécules d'acides nucléique de séquence SEQ ID NO : 4, SEQ ID NO : 8 et SEQ ID NO : 9.

Afin de s'affranchir des variabilités liées aux expérimentations, le niveau d'expression de chacun des gènes d'intérêt de l'invention, c'est-à-dire au moins les gènes de séquence SEQ ID NO : 1 à 7, et de leur variants quand ils existent, choisis parmi les gènes de séquence SEQ ID NO : 1 à 25, est normalisé par rapport au niveau d'expression d'un ou plusieurs gènes dont le niveau d'expression n'est pas modulé (augmenté ou diminué) dans le cadre de la leucémie myéloïde chronique, ou plus généralement dans la cadre d'une quelconque pathologie.

Le ou les gènes permettant la normalisation sont communément les gènes dits « de ménage » (« housekeeping genes » en anglais), qui correspondent à des gènes codant des protéines participant à l'architecture des cellules, comme l'actine ou la tubuline, ou encore des gènes codant des enzymes du métabolisme, comme par exemple le gène GAPDH codant la glycéraldéhyde-3-phosphate déshydrogénase.

Ainsi en pratique, pour un gène donné, on mesure son niveau d'expression et on obtient une valeur 1. En parallèle, on mesure le niveau d'expression de la GAPDH, et l'on obtient un score 2.

Le niveau d'expression normalisé dudit gène donné, lorsque l'on utilise par exemple la technique de northern blot, est alors obtenu par le ratio suivant : score1/score2.

Comme mentionné précédemment, le niveau d'expression des gènes étudiés dans la méthode de pronostic susmentionnée est comparé au niveau d'expression des mêmes gènes issus d'échantillons biologiques issus d'individus sains. Le niveau d'expression des gènes issus desdits échantillons biologiques sains est également normalisé par rapport à un ou plusieurs gènes « de ménage ».

Aussi, le ratio i tel que défini dans la formule 1 peut être redéfini de la manière suivante :

$$\text{Ratio i} = \frac{\dfrac{\text{score1 i (patient)}}{\text{score2 (patient)}}}{\dfrac{score1\ i(sain)}{score2\ (sain)}}$$

où

- score1 i (patient) est le niveau d'expression mesuré pour le gène i dans l'échantillon issu de patient,
- score2 (patient) est le niveau d'expression mesuré pour le gène de ménage dans l'échantillon issu de patient,
- score1 i (sain) est le niveau d'expression mesuré pour le gène i dans l'échantillon issu d'un individu sain,
- score2 (sain) est le niveau d'expression mesuré pour le gène de ménage dans l'échantillon issu d'un individu sain.

Ainsi, la formule 1 peut se réécrire de la manière suivante :

$$S = \sum \frac{\frac{\text{score1 i (patient)}}{\text{score2 (patient)}}}{\frac{score1\ i(sain)}{score2\ (sain)}} - \sum \frac{\frac{\text{score1 j (patient)}}{\text{score2 (patient)}}}{\frac{score1\ j(sain)}{score2\ (sain)}}$$

où i et j sont des entiers, i variant de 1 à 5 et j variant de 6 à 7. Une autre formulation serait la suivante :

$$S = \left( \frac{\frac{\text{score1 1 (patient)}}{\text{score2 (patient)}}}{\frac{score1\ 1(sain)}{score2\ (sain)}} + \frac{\frac{\text{score1 2 (patient)}}{\text{score2 (patient)}}}{\frac{score1\ 2(sain)}{score2\ (sain)}} + \frac{\frac{\text{score1 3 (patient)}}{\text{score2 (patient)}}}{\frac{score1\ 3(sain)}{score2\ (sain)}} + \frac{\frac{\text{score1 4 (patient)}}{\text{score2 (patient)}}}{\frac{score1\ 4(sain)}{score2\ (sain)}} + \frac{\frac{\text{score1 5 (patient)}}{\text{score2 (patient)}}}{\frac{score1\ 5\ (sain)}{score2\ (sain)}} \right) - \left( \frac{\frac{\text{score1 6 (patient)}}{\text{score2 (patient)}}}{\frac{score1\ 6(sain)}{score2\ (sain)}} + \frac{\frac{\text{score1 7 (patient)}}{\text{score2 (patient)}}}{\frac{score1\ 7(sain)}{score2\ (sain)}} \right)$$

Dans le cas particulier de l'utilisation d'une méthode quantitative de mesure de l'expression des gènes, et notamment par PCR quantitative, on utilisera pour chaque gène deux échantillons indépendants, et le ratio sera calculé par le $2^{-\Delta\Delta Ct}$ où $\Delta\Delta Ct = \Delta Ct$échantillon1 - $\Delta Ct$échantillon2 et $\Delta Ct$ = Ct ARN - Ct ARN de référence (voir ci-après).

Une autre formulation sera alors la suivante :

$$S = \sum 2^{-\Delta\Delta \text{Ct i}} - \sum 2^{-\Delta\Delta \text{Ct j}}$$

où i varie de 1 à 5 et j varie de 6 à 7, soit

$$S = \left(2^{-\Delta\Delta \text{Ct1}} + 2^{-\Delta\Delta \text{Ct2}} + 2^{-\Delta\Delta \text{Ct3}} + 2^{-\Delta\Delta \text{Ct4}} + 2^{-\Delta\Delta \text{Ct5}}\right) - \left(2^{-\Delta\Delta \text{Ct6}} + 2^{-\Delta\Delta \text{Ct7}}\right).$$

Dans un mode de réalisation avantageux, l'invention concerne une méthode de pronostic *in vitro* telle que définie précédemment, dans laquelle

- si S est supérieur ou égal à 1 et inférieur ou égal à 2, ledit individu aura de 40% à 10% de chances de présenter une rémission moléculaire majeure un an après le début d'une thérapie avec un inhibiteur de tyrosine kinase de première génération.

Avantageusement, il est possible d'affiner le pronostic du patient et de déterminer quelle sera sa réponse moléculaire majeur après un an de traitement avec un inhibiteur de tyrosine kinase de première génération, notamment l'imatinib mesylate.

Ainsi, si :

- le score S est inférieur à 1, ledit individu aura au moins environ 40% de chances après 1 an de présenter une rémission moléculaire majeure s'il est traité avec un inhibiteur de tyrosine kinase de première génération,
- le score S est supérieur à 1 mais inférieur ou égal à 2, ledit individu aura de 40% à 10% de chances après 1 an de présenter une rémission moléculaire majeure s'il est traité avec un inhibiteur de tyrosine kinase de première génération.

Cela signifie que plus le score S augmente, plus le risque du patient de développer une résistance à l'inhibiteur de tyrosine kinase de première génération est grand, ou, en d'autres termes, plus les chances après 1 an de présenter une rémission moléculaire majeure diminuent.

Plus avantageusement, l'invention concerne une méthode de pronostic in vitro telle que définie précédemment, dans laquelle :

- si S supérieur à 2, ledit individu aura moins de 10% de chances de présenter une rémission moléculaire majeure un an après le début d'une thérapie avec un inhibiteur de tyrosine kinase de première génération.

En d'autres termes, selon ce mode de réalisation avantageux, l'invention concerne une méthode de pronostic in vitro telle que définie précédemment, dans laquelle, si :

- le score S est inférieur à 1, ledit individu aura au moins environ 40% de chances après 1 an de présenter une rémission moléculaire majeure s'il est traité avec un inhibiteur de tyrosine kinase de première génération,
- le score S est supérieur à 1 mais inférieur ou égal à 2, ledit individu aura de 40% à 10% de chances après 1 an de présenter une rémission moléculaire majeure s'il est traité avec un inhibiteur de tyrosine kinase de première génération, et
- S supérieur à 2, ledit individu aura moins de 10% de chances après 1 an de présenter une rémission moléculaire majeure s'il est traité s'il est traité avec un inhibiteur de tyrosine kinase de première génération.

L'inventeur a constaté de manière surprenante que le niveau d'expression d'au moins les 7 gènes de séquence SEQ ID NO : 1 à 7 variait selon le pronostic des patients traités avec un inhibiteur de tyrosine kinase de première génération.

Ainsi, l'invention peut être avantageusement définie comme une méthode de pronostic in vitro de la réponse à une thérapie d'un individu atteint d'une leucémie myéloïde chronique, à partir d'un échantillon biologique leucémique issu dudit individu, ladite méthode comprenant :

a. une étape de mesure du niveau d'expression des gènes d'au moins un sous-groupe de gènes choisis dans un groupe de gènes,
ledit groupe de gènes étant constitué de 25 gènes, lesdits 25 gènes comprenant ou étant constitués par les séquences d'acides nucléiques SEQ ID NO : 1 à SEQ ID NO : 25,
ledit sous-groupe consistant en 7 gènes, lesdits 7 gènes comprenant ou étant constitués par les séquences d'acides nucléiques SEQ ID NO : 1 à SEQ ID NO : 7,
une valeur du niveau d'expression mesuré étant obtenue pour chacun des gènes dudit sous-groupe,
b. une étape de comparaison de la valeur attribuée à l'étape précédente à chacun desdits gènes dudit sous-groupe à la valeur attribuée à chacun desdits gènes dudit sous-groupe obtenue à partir d'un échantillon biologique sain, afin d'obtenir un ratio pour chacun desdits gènes dudit sous-groupe du niveau d'expression dans l'échantillon biologique leucémique sur le niveau d'expression dans l'échantillon sain, et
c. une étape de détermination d'un score S selon la formule suivante

$$S = \sum ratio\ i - \sum ratio\ j$$

où ratio i et ratio j représentent respectivement les ratios obtenus pour lesdits gènes dudit sous-groupe comprenant ou étant constitués par les séquences d'acides nucléiques SEQ ID NO : i ou SEQ ID NO : j,
où i et j sont des entiers, i variant de 1 à 5 et j variant de 6 à 7,
de sorte que :

- si le score S est inférieur à 1, ledit individu aura au moins environ 40% de chances après 1 an de présenter une rémission moléculaire majeure s'il est traité avec un inhibiteur de tyrosine kinase de première génération,
- si le score S est supérieur à 1 mais inférieur ou égal à 2, ledit individu aura de 40% à 10% de chances après 1 an de présenter une rémission moléculaire majeure s'il est traité avec un inhibiteur de tyrosine kinase de première génération, et

- si S supérieur à 2, ledit individu aura moins de 10% de chances après 1 an de présenter une rémission moléculaire majeure s'il est traité s'il est traité avec un inhibiteur de tyrosine kinase de première génération.

Encore plus avantageusement, l'invention concerne la méthode susmentionnée dans laquelle :

- si le score S est inférieur à 1, ledit individu aura
  - au moins environ 40% de chances après 1 an de présenter une rémission moléculaire majeure s'il est traité avec un inhibiteur de tyrosine kinase de première génération, et
  - moins d'environ 60% de chances après 1 an de présenter une rémission moléculaire intermédiaire s'il est traité avec un inhibiteur de tyrosine kinase de première génération,
- si le score S est supérieur à 1 mais inférieur ou égal à 2, ledit individu aura
  - jusqu'à 40% de chances après 1 an de présenter une rémission moléculaire intermédiaire s'il est traité avec un inhibiteur de tyrosine kinase de première génération, et
  - jusqu'à environ 25% de chances après 1 an d'être mauvais répondeur s'il est traité avec un inhibiteur de tyrosine kinase de première génération,

  et
- si S supérieur à 2, ledit individu aura
  - moins de 10% de chances après 1 an de présenter une rémission moléculaire majeure s'il est traité avec un inhibiteur de tyrosine kinase de première génération,
  - jusqu'à environ 40% de chances après 1 an de présenter une rémission moléculaire intermédiaire s'il est traité avec un inhibiteur de tyrosine kinase de première génération, et
  - environ 50% ou plus de chances d'être mauvais répondeur s'il est traité avec un inhibiteur de tyrosine kinase de première génération.

On entend dans l'invention par « réponse moléculaire intermédiaire » la quantité de transcrits BCR-ABL dans les cellules de niveau intermédiaire, et avantageusement un ratio BCR-ABL/ABL non recombiné compris entre 0,1% et 10% inclus (]0,1%-10%]).

On entend dans l'invention par «mauvais répondeur», un patient dont la quantité de transcrits BCR-ABL dans les cellules est de niveau intermédiaire, et avantageusement avec un ratio BCR-ABL/ABL non recombiné supérieur à 10%.

Dans un autre mode de réalisation, l'invention concerne une méthode de pronostic *in vitro* telle que définie précédemment, dans laquelle la valeur du niveau d'expression mesuré est obtenue par une mesure de l'expression desdits gènes du sous-groupe par une méthode de mesure quantitative, notamment la méthode de PCR quantitative.

Afin de mesurer le niveau d'expression des gènes d'intérêt, il est possible d'utiliser différentes techniques connues de l'homme du métier :

- le northern-blot, est une méthode de biologie moléculaire permettant l'analyse de l'ARN. Elle dérive du Southern blot sauf qu'au lieu d'étudier de l'ADN, on étudie de l'ARN. L'ARN va être analysé par électrophorèse, permettant de séparer les ARN en fonction de leur taille. Puis ils sont détectés par une sonde d'ADN ou d'ARN. Le northern-blot permet d'apprécier la distribution des ARN dans les tissus et d'étudier leur abondance relative. On peut alors déduire de ces observations l'expression plus ou moins importante de certains gènes. L'utilisation de marqueurs radioactifs ou fluorescents permet de quantifier le niveau d'expression.
- Les puces à ADN : le principe des puces à ADN, dont l'utilisation s'est répandue depuis les années 1990, est apparenté au northern puisqu'il est fondé sur la fixation sur un support de fragments isolés d'ADN rétrostranscrits et l'hybridation avec une sonde faite à partir d'ADN.
- La PCR - quantitative : Le principe de la PCR quantitative dite « en temps réel » repose sur la possibilité de suivre la quantité d'ADN présente dans la réaction à tout instant et non à la fin de la PCR (PCR point final). Des sondes fluorescentes se fixent soit sur l'ADN double brin (technologie SYBR) soit sur une séquence d'ADN précise (technologie Taqman et Beacon). Ces sondes ne fluorescent qu'une fois fixées à l'ADN (soit à cause d'un "quencher" soit car la fluorescence nécessite un ADN double brin). Un seuil de fluorescence est établi par le programme de la machine de PCR en temps réel. Une fois que la quantité d'ADN permet aux sondes fluorescentes de dépasser ce seuil alors on obtient un numéro de cycle PCR appelé "Ct" pour "Cycle Threshold" ou cycle seuil. C'est cette valeur qui est à la base des calculs pour quantifier l'ADN de façon absolue ou relative. Il est important de connaître l'efficacité E de la PCR. Pour cela, on effectue une PCR en temps réel sur des échantillons de dilution croissante pour obtenir une courbe étalon correspondant au couple d'amorces utilisé (spécifiques du locus d'intérêt). Par exemple, une série de dilution au 1/2 ($D_{n+1}=D_n/2$) doit, en théorie, donner des courbes d'amplification décalées d'un cycle PCR à chaque fois. Si tel est le cas, la réaction a alors une efficacité égale à 2 (la quantité d'ADN double à chaque cycle). En pratique, le programme de la machine de PCR en temps réel peut calculer l'efficacité E de la réaction. Plus souvent, une PCR en temps réel sur une série de dilution avec une quantité d'ADN initiale connue permet de calculer l'efficacité de la réaction. Les Ct sont placés sur un graphe en échelle logarithmique et l'équation de la régression linéaire passant par ces points donne l'efficacité (c'est le coefficient directeur).

Pour des raisons pratiques et de spécificité, il sera avantageux dans le cadre de l'invention d'utiliser la PCR quantitative utilisant la technologie Taqman et Beacon : l'expression d'un gène est suivie par une amplification par un couple d'amorce spécifiques, et la présence d'une sonde quencher permettant de quantifier le nombre de molécules.

Plus avantageusement, l'invention concerne une méthode de pronostic in vitro telle que définie précédemment, dans laquelle la valeur du niveau d'expression mesuré est obtenue par une mesure de l'expression desdits gènes du sous-groupe mise en œuvre en utilisant au moins les oligonucléotides comprenant ou constitués des séquences SEQ ID : 32 à 45, notamment les oligonucléotides comprenant ou constitués des séquences SEQ ID : 32 à 45 et les séquences suivantes : 5'-tggggaag-3', 5'-ctgctggg-3', 5'-tgctggag-3', 5'-ggtggtgg-3', 5'-caggagaa-3', 5'-ctgcccca-3' et 5'-ctggctgg-3'.

Dans l'invention, il est avantageux de déterminer le niveau d'expression desdits 7 gènes de séquence SEQ ID NO : 1 à 7, et des variants envisagés ci-dessus, représentés par les séquences SEQ ID NO: 26 à 31, avec les oligonucléotides suivants :

| Gène | Oligonucléotide sens | Oligonucléotide antisens | Sonde TaqMan® |
|---|---|---|---|
| CAT | 5'-cgcagttcggttctccac-3' SEQ ID NO : 32 | 5'-gggtcccgaactgtgtca-3' SEQ ID NO : 33 | 5'-tgctggag-3' |
| SOD1 | 5'-gcatcatcaatttcgagcag-3' SEQ ID NO : 34 | 5'-caggccttcagtcagtcctt-3' SEQ ID NO : 35 | 5'-tggggaag-3' |
| GPX1 | 5'-caaccagtttgggcatcag-3' SEQ ID NO : 36 | 5'-gttcacctcgcacttctcg-3' SEQ ID NO : 37 | 5'-ggtggtgg-3' |
| GPX4 (1-2-3) | tacggacccatggaggag-3' SEQ ID NO : 38 | 5'-ccacacacttgtggagctagaa-3' SEQ ID NO : 39 | 5'-ctgcccca-3' |
| PRDX1 | 5'-cactgacaaacatggggaagt-3' SEQ ID NO : 40 | 5'-tttgctcttttggacatcagg-3' SEQ ID NO : 41 | 5'-ctggctgg-3' |
| SOD2 (1-2-3) | 5'-tccactgcaaggaacaacag-3' SEQ ID NO : 42 | 5'-taagcgtgctcccacacat-3' SEQ ID NO : 43 | 5'-ctgctggg-3' |
| GPX2 | 5'-gtccttggcttcccttgc-3' SEQ ID NO : 44 | 5'-tgttcaggatctcctcattctg-3' SEQ ID NO : 45 | 5'-caggagaa-3' |

En d'autres termes, ce mode de réalisation avantageux concerne une méthode de pronostic in vitro telle que définie précédemment, dans laquelle la valeur du niveau d'expression mesuré est obtenue par une mesure de l'expression desdits gènes du sous-groupe mise en œuvre en utilisant au moins les oligonucléotides comprenant ou constitués des séquences SEQ ID : 32 à 45, tels que

- Les oligonucléotides SEQ ID NO : 32 et 33 permettent de mesurer l'expression du gène de séquence SEQ ID NO : 1
- Les oligonucléotides SEQ ID NO : 34 et 35 permettent de mesurer l'expression du gène de séquence SEQ ID NO : 2
- Les oligonucléotides SEQ ID NO : 36 et 37 permettent de mesurer l'expression du gène de séquence SEQ ID NO : 3
- Les oligonucléotides SEQ ID NO : 38 et 39 permettent de mesurer l'expression du gène de séquence SEQ ID NO : 4
- Les oligonucléotides SEQ ID NO : 40 et 41 permettent de mesurer l'expression du gène de séquence SEQ ID NO : 5
- Les oligonucléotides SEQ ID NO : 42 et 43 permettent de mesurer l'expression du gène de séquence SEQ ID NO : 6, et
- Les oligonucléotides SEQ ID NO : 44 et 45 permettent de mesurer l'expression du gène de séquence SEQ ID NO : 7, et

notamment dans laquelle la valeur du niveau d'expression mesuré est obtenue par une mesure de l'expression desdits gènes du sous-groupe mise en œuvre en utilisant au moins les oligonucléotides comprenant ou constitués des séquences SEQ ID : 32 à 45, tels que

- les oligonucléotides SEQ ID NO : 32, SEQ ID NO : 33 et 5'-tggggaag-3' permettent de mesurer l'expression du gène de séquence SEQ ID NO : 1
- les oligonucléotides SEQ ID NO : 34, SEQ ID NO : 35 et 5'-ctgctggg-3' permettent de mesurer l'expression du gène de séquence SEQ ID NO : 2
- les oligonucléotides SEQ ID NO : 36, SEQ ID NO : 37 et 5'-tgctggag-3' permettent de mesurer l'expression du gène de séquence SEQ ID NO : 3
- les oligonucléotides SEQ ID NO : 38, SEQ ID NO : 39 et 5'-ggtggtgg-3' permettent de mesurer l'expression du gène de séquence SEQ ID NO : 4
- les oligonucléotides SEQ ID NO : 40, SEQ ID NO : 41 et 5'-caggagaa-3' permettent de mesurer l'expression du gène de séquence SEQ ID NO : 5
- les oligonucléotides SEQ ID NO : 42, SEQ ID NO : 43 et 5'-ctgcccca-3'permettent de mesurer l'expression du gène de séquence SEQ ID NO : 6, et
- les oligonucléotides SEQ ID NO : 44, SEQ ID NO : 45 et 5'-ctggctgg-3' permettent de mesurer l'expression du gène de séquence SEQ ID NO : 7.

Il est en outre avantageux de normaliser l'expression de chacun des gènes susmentionnés avec l'expression de la GAPDH en utilisant les oligonucléotides de séquence SEQ ID NO : 46 et 47, et de séquence 5'-tggggaag-3'.

Avantageusement, l'invention concerne une méthode de pronostic *in vitro* telle que définie précédemment, où la valeur du niveau d'expression mesuré est obtenue par une mesure de l'expression desdits gènes du sous-groupe, ladite mesure utilisant les oligonucléotides suivants :

- les oligonucléotides SEQ ID NO : 32 et 33 pour mesurer l'expression du gène comprenant ou étant constitué par la séquence d'acides nucléiques SEQ ID NO : 1,
- les oligonucléotides SEQ ID NO : 34 et 35 pour mesurer l'expression du gène comprenant ou étant constitué par la séquence d'acides nucléiques SEQ ID NO : 2
- les oligonucléotides SEQ ID NO : 36 et 37 pour mesurer l'expression du gène comprenant ou étant constitué par la séquence d'acides nucléiques SEQ ID NO : 3
- les oligonucléotides SEQ ID NO : 38 et 39 pour mesurer l'expression du gène comprenant ou étant constitué par la séquence d'acides nucléiques SEQ ID NO : 4
- les oligonucléotides SEQ ID NO : 40 et 41 pour mesurer l'expression du gène comprenant ou étant constitué par la séquence d'acides nucléiques SEQ ID NO : 5
- les oligonucléotides SEQ ID NO : 42 et 43 pour mesurer l'expression du gène comprenant ou étant constitué par la séquence d'acides nucléiques SEQ ID NO : 6, et
- les oligonucléotides SEQ ID NO : 44 et 45 pour mesurer l'expression du gène comprenant ou étant constitué par la séquence d'acides nucléiques SEQ ID NO : 7.

L'invention concerne en outre une méthode de diagnostic personnalisé ou théranostic *in vitro,* d'un individu atteint d'une leucémie myéloïde chronique, comprenant

b. une étape de mesure du niveau d'expression d'au moins un sous-groupe de gènes choisis dans un groupe de gènes,
ledit groupe de gènes étant constitué de 25 gènes, lesdits 25 gènes comprenant ou étant constitués par les séquences d'acides nucléiques SEQ ID NO : 1 à SEQ ID NO : 25,
ledit sous-groupe consistant en 7 gènes, lesdits 7 gènes comprenant ou étant constitués par les séquences d'acides nucléiques SEQ ID NO : 1 à SEQ ID NO : 7,
une valeur du niveau d'expression mesuré étant obtenue pour chacun des gènes dudit sous-groupe,
c. une étape de comparaison de la valeur attribuée à l'étape précédente à chacun desdits gènes dudit sous-groupe à la valeur attribuée à chacun desdits gènes dudit sous-groupe obtenue à partir d'un échantillon biologique sain, afin d'obtenir un ratio pour chacun desdits gènes dudit sous-groupe du niveau d'expression dans l'échantillon biologique leucémique sur le niveau d'expression dans l'échantillon sain, et
d. une étape de détermination d'un score S selon la formule suivante

$$S = \sum ratio\ i - \sum ratio\ j$$

où ratio i et ratio j représentent respectivement les ratios obtenus pour lesdits gènes dudit sous-groupe comprenant ou étant constitués par les séquences d'acides nucléiques SEQ ID NO : i ou SEQ ID NO : j,
où i et j sont des entiers, i variant de 1 à 5 et j variant de 6 à 7,
de sorte que

- si S est inférieur à 1, la leucémie myéloïde chronique dudit individu est une leucémie myéloïde chronique susceptible de répondre préférentiellement à un traitement comprenant un inhibiteur de tyrosine kinase de première génération, et
- si S est supérieur à 2, la leucémie myéloïde chronique dudit individu est une leucémie myéloïde chronique susceptible de répondre préférentiellement à un traitement comprenant un inhibiteur de tyrosine kinase de seconde génération ou de génération ultérieure.

Dans cette méthode de diagnostic personnalisé, notamment in vitro, de l'invention, il est possible de déterminer quel type de traitement il sera avantageux de fournir au patient atteint de leucémie myéloïde chronique afin d'obtenir une réponse moléculaire majeure.

L'inventeur a fait la constatation surprenante selon laquelle lorsque que l'on mesure le niveau d'expression des gènes appartenant au groupe de gènes représentés par les acides nucléiques de séquences SEQ ID NO : 1 à SEQ ID NO : 7, et que l'on applique la formule 1 susmentionnée, il est possible de déterminer quel sera le meilleur traitement à proposer au patient. En effet, lorsque S, tel que calculé comme indiqué précédemment, est

- inférieur ou égal à 1, le patient aura plus de 40% de chances de présenter une réponse moléculaire majeure à 1an après le début du traitement s'il est traité avec un inhibiteur de tyrosine kinase de première génération, notamment avec de l'imatinib mesylate, Le traitement à l'imatinib mesylate est donc recommandé et approprié pour le patient.
- supérieur ou égal à 2, le patient aura moins de 10% de chances de présenter une réponse moléculaire majeure à 1an après le début du traitement s'il est traité avec un inhibiteur de tyrosine kinase de première génération, notamment avec de l'imatinib mesylate. Il sera donc approprié de proposer au patient un autre traitement et notamment de lui proposer un traitement à base d'inhibiteur de tyrosine kinase de seconde génération ou de génération ultérieure.

Dans le cas particulier où 1<S<2, le praticien est confronté à un choix entre le traitement avec un inhibiteur de première génération et de seconde génération ou de génération(s) ultérieure(s). Dans cette situation, le praticien s'aidera alors des éléments cliniques, des co-morbidités en lien avec les effets secondaires potentiels et des scores pronostic clinico-biologiques (Score de Sokal...).

Avantageusement, l'invention concerne une méthode telle que définie précédemment, dans laquelle l'inhibiteur de tyrosine kinase de première génération est l'imatinib ou l'un de ses sels.

L'imatinib mésylate est un inhibiteur compétitif sélectif et puissant de l'ATP pour son site de liaison sur abl, induisant une inhibition de l'activité tyrosine kinase. C'est aujourd'hui le traitement de première intention. Il est utilisé à la dose de 400 mg/j per os. Il permet d'obtenir des réponses hématologiques classiquement en un à trois mois.

En absence de réponse hématologique complète à 3 mois, on parlera d'échec de traitement. Le taux de rémission hématologique à 5 ans est de plus de 98% et le taux de rémission complète cytogénétique à 5 ans est supérieur à 87%.

Les effets secondaires sont fréquents mais d'intensité modérée: nausées, diarrhée, crampes, œdèmes et éruptions cutanées. Ils sont exacerbés chez les sujets âgés. Cependant, ils conduisent rarement à l'arrêt du traitement.

En raison du risque de neutropénie et de thrombopénie, une surveillance régulière de l'hémogramme doit être pratiquée tous les quinze jours pendant les 3 premiers mois.

Il existe des résistances dites primaires et d'autres secondaires (acquises).

Les résistances primaires sont définies comme une situation de non réponse sur le plan hématologique, cytogénétique et moléculaire après des durées de traitement précises. Celles-ci impliquent alors de proposer d'autres alternatives de traitement ou d'augmenter les doses.

Tandis que les résistances secondaires sont définies par une perte de la réponse initiale ou par transformation. Plusieurs mécanismes de résistance ont été mis en évidence: modification de la biodisponibilité intracellulaire de l'imatinib, sur-expression du gène MDR (multidrug resistance), amplification de BCR-ABL, mutations du domaine kinase d'abl (>50 mutations différentes), mécanismes BCR-ABL indépendants. Dans ces cas, une augmentation de la posologie peut parfois être efficace (600 voire 800mg/j) ou un changement vers un inhibiteur de tyrosine kinase (ITK) dit de deuxième génération ou de générations ultérieures.

Dans un autre mode de réalisation avantageux, l'invention concerne une méthode telle que définie ci-dessus, dans laquelle l'inhibiteur de tyrosine kinase de seconde génération est le dasatinib ou le nilotinib, ou l'un de leurs sels.

Le dasatinib (Sprycel®) est un ITK dit de 2ème génération, ayant montré son efficacité chez des patients intolérants ou ayant des mécanismes de résistance par mutations acquises au Glivec®. Il est utilisé à la dose de 100mg/j. En plus de bloquer l'activité kinase de BCR-ABL, il inhibe d'autres voies de transduction comme les kinases de la famille des SRC. Contrairement à l'imatinib, le dasatinib se lie à la fois au domaine kinase de ABL dans sa conformation active et inactive, ce qui explique sa grande efficacité. La plupart des mutations de sensibilité diminuée à l'imatinib sont sensibles au dasatinib, à l'exception d'une mutation du site ATP d'ABL, associée à une résistance complète (Thréonine en Isoleucine au niveau du codon 315, T315I).

Plus récemment, cette molécule a obtenu une AMM pour une utilisation en deuxième ligne.

Parmi les effets indésirables, on note surtout des rétentions hydriques (épanchements pleuraux, péricardiques), une toxicité hématologique, diarrhée. Ainsi cette molécule est à éviter en cas d'antécédents d'hypertension, de cardiopathie ou de maladie respiratoire. Quelques cas d'hypertension artérielle pulmonaire ont été rapportés.

Le nilotinib (Tasigna®) est, comme le dasatinib, un ITK dit de 2$^{ème}$ génération, ayant montré son efficacité chez des patients intolérants ou ayant des mécanismes de résistance par mutations acquises au Glivec®. Il s'agit d'un analogue de l'imatinib plus puissant. Il peut cibler également d'autre protéine kinase comme les récepteurs c-Kit. Comme l'imatinib, le nilotinib se lie à la conformation inactive de la kinase. Il est préconisé à la dose de 400mg 2 fois par jour à 12h d'intervalle, et à distance des repas. Les aliments entrainent une modification de sa biodisponibilité.

Le nilotinib est bien toléré, les principaux effets indésirables sont l'augmentation de la lipase, de la bilirubine, de la glycémie, une hypophosphatémie, une toxicité cutanée ainsi qu'une toxicité hématologique moins importante que le dasatinib. On évitera d'utiliser le nilotinib chez les patients ayant des antécédents de pancréatite ou de diabète mal équilibré, ainsi que les patients présentant des artériopathies

Les inhibiteurs de tyrosine kinase de génération ultérieure sont notamment : le bosutinib, le ponatinib ou des composés en phase de développement clinique, tel que le bafetinib.

Il est en outre décrit un kit, ou trousse, comprenant,

a. au moins les oligonucléotides comprenant ou constitués des séquences SEQ ID : 32 à 45, en particulier les oligonucléotides comprenant ou constitués des séquences SEQ ID : 32 à 45 ainsi que les oligonucléotides constitués des séquences suivantes : 5'-tggggaag-3', 5'-ctgctggg-3', 5'-tgctggag-3', 5'-ggtggtgg-3', 5'-caggagaa-3', 5'-ctgcccca-3' et 5'-ctggctgg-3', et

b. les acides nucléiques d'un ou plusieurs échantillons biologiques sains, notamment les acides nucléiques d'un ou plusieurs échantillons de cellules polynucléaires ou neutrophiles du sang périphérique d'un ou plusieurs individus non leucémiques, c'est-à-dire d'un ou plusieurs individus n'étant pas affecté par une leucémie.

L'échantillon biologique sain est tel qu'il est défini précédemment.

Avantageusement, le kit susdécrit, ou la trousse, comprend :

a. au moins les oligonucléotides comprenant ou constitués des séquences SEQ ID : 32 à 45, en particulier les oligonucléotides comprenant ou constitués des séquences SEQ ID : 32 à 45 ainsi que les oligonucléotides constitués des séquences suivantes : 5'-tggggaag-3', 5'-ctgctggg-3', 5'-tgctggag-3', 5'-ggtggtgg-3', 5'-caggagaa-3', 5'-ctgcccca-3', 5'-ctggctgg-3', 5'-ctggctgg-3' et 5'-tggggaag-3', et

b. les acides nucléiques d'un échantillon biologique sain, notamment les acides nucléiques d'un échantillon de cellules polynucléaires ou neutrophiles du sang périphérique d'un individu non leucémique.

Les acides nucléiques de l'échantillon sain sont avantageusement des ARNs conservés dans des conditions limitant leur dégradation.

La trousse ou le kit peut en outre contenir des instructions sur un support approprié permettant de mettre en oeuvre la méthode de diagnostic ou de théranostic susmentionnées. Il peut s'agir par exemple d'instructions définissant le programme de PCR quantitative (nombre de cycles, températures etc...), et aussi un produit programme d'ordinateur sur un support approprié permettant de réaliser le calcul de S, à l'aide de la formule susmentionnée.

Un autre aspect de l'invention porte sur un logiciel ou un produit programme d'ordinateur conçu pour mettre en œuvre la méthode susmentionnée et/ou comprenant des portions/moyens/instructions de code de programme pour l'exécution de ladite méthode lorsque ledit programme est exécuté sur un ordinateur. Avantageusement ledit programme est compris dans un support d'enregistrement de données lisible par ordinateur. Un tel support n'est pas limité à un support d'enregistrement portable tel qu'un CD-ROM mais peut également faire part d'un dispositif comprenant une mémoire interne dans un ordinateur (par exemple des RAM et/ou ROM), ou de dispositif à mémoires externes tels des disques durs ou des clefs USB, ou un serveur à proximité ou à distance.

Avantageusement, l'invention concerne le produit programme d'ordinateur susmentionné, pour la mise en œuvre des étapes b et c de la méthode de pronostic telle que définie précédemment.

L'invention sera mieux comprise à la lumière des quatre figures et de l'exemple suivants.

## Brève description des figures

La **figure 1** représente un graphique montrant le niveau d'expression des 25 gènes testés chez les 35 patients testés. Le niveau contrôle (correspondant à l'expression des gènes dans des échantillons sains est normalisé à 1. L'échelle est logarithmique.

Les gènes sont les suivants : #1 : SOD2, #2 : GLRX1(1-2), #3 : GSR, #4 : PRDX5(1-3), #5 : PRDX3(1-3), #6 : TXN, #7, CAT, #8 : SOD1, #9, GPX1(1), #10 : GPX4(1-2-3), #11 : PRDX2(1), #12 : PRDX1 (1-2-3), #13 : GPX1(2), #14 : GPX3, #15 : GPX7, #16 : TXN2, #17 : PRDX5(2), #18 : GLRX2(2), #19 : GLRX5, #20 : PRDX2(3), #21 : PRDX4, #22 : GLRX3, #23 : PRDX6, #24 : GPX2 et #25 : GLRX2(1).

La **figure 2** représente un graphique montrant le niveau d'expression des 25 gènes testés chez les 35 patients, et regroupés selon leur réponse moléculaire : A : réponse moléculaire majeure, B : réponse moléculaire intermédiaire et C : mauvais répondeurs.

La **figure 3** est un histogramme montrant la répartition en pourcentage des types des réponses moléculaire selon le score S calculé. Les régions en noir correspondent à une réponse moléculaire majeure, les régions en hachuré correspondent à une réponse moléculaire intermédiaire, et les régions grises correspondent aux mauvais répondeurs.

La **figure 4** est un graphique représentant les valeurs du score S (ordonnée) en fonction de la distribution des patients selon la réponse moléculaire à l'imatinib mesylate obtenue un an après le diagnostic (en abscisse de gauche à droite : majeure, intermédiaire ou non répondeur).

## EXEMPLE :

### Matériel et Méthodes

#### 1- Isolement des polynucléaires sanguins

Les polynucléaires du sang périphérique sont isolés par centrifugation sur Ficoll de densité d=1,077.

#### 2- Extraction des ARN

Les ARN sont extraits à partir de $5.10^6$ cellules. Ces dernières ont été lavées deux fois en PBS, suivit de l'ajout de 1 mL de Trizol® (Invitrogen). Les tubes sont agités (vortex®) pendant 15 min pour bien lyser les cellules. 200 $\mu$L de chloroforme sont ajoutés pour obtenir 3 phases, suite à un passage au vortex tube par tube pendant 45 sec et à une centrifugation de 15 min à 12000 g à 4°C. La phase aqueuse supérieure contenant les ARN, la phase intermédiaire renfermant les protéines et la phase inférieure correspondant au chloroforme et phénol. Une deuxième extraction au chloroforme est réalisée sur la phase supérieure contenant les ARN. Puis, 500 $\mu$L d'isopropanol sont ajoutés à la phase supérieure de la deuxième extraction au chloroforme. Il est nécessaire de retourner les tube une dizaine de fois pour que l'isopropanol précipite les ARN, puis ces derniers sont centrifugés 10 min à 12000 g. Un premier lavage des ARN est effectué sur le culot du tube auquel est ajouté 1 mL d'éthanol 75 % suivant une centrifugation de 5 min à 7500 g. Un deuxième lavage est réalisé avec 500 $\mu$L d'éthanol 75 %. Le surnageant est éliminé une nouvelle fois par retournement et les tubes sont déposés à l'envers sur des compresses pendant au moins 20 min pour que tout l'éthanol s'évapore. L'ARN est ensuite dissout dans 40 μL d'eau DEPC et les tubes ont été mis 1 h à -20°C pour une meilleure dissolution des ARN.

**3- Dosage des ARN, analyse de leur pureté et de leur qualité**

La concentration des ARN est évaluée par la lecture de l'absorbance à 260 nm à l'aide du spectrophotomètre NanoDrop®. La détermination de la contamination des ARN s'effectue en faisant le rapport de l'absorbance à 260 nm sur celle à 280 nm. L'ARN est considéré comme exempt de contamination pour un rapport A260/A280 compris entre 1,9 et 2,1. La qualité des ARN est ensuite vérifiée par Bioanalyzer selon les recommandations du fabricant.

**4- Reverse Transcription (RT)** :

La transcription inverse (Reverse Transcription, RT) est la réaction de synthèse d'un brin d'ADN avec pour matrice un brin d'ARN. La transcriptase inverse est une ADN polymérase ARN dépendante synthétisant un brin d'ADN dit complémentaire (ADNc) d'un brin d'ARN. De plus, cette enzyme ne peut synthétiser l'ADNc qu'à partir d'une zone double brin crée par l'hybridation de l'ARN avec une amorce.

La RT est réalisée avec le kit « SuperScript® VILO™ cDNA synthesis kit » (Invitrogen). Une réaction est réalisée avec un tube de 5 μg d'ARN précédemment aliquoté. L'incubation des tubes 10 min à 70°C permet de linéariser l'ARN pour une meilleure synthèse de l'ADNc. Puis, 50 μL du mélange réactionnel suivant sont ajoutés à chaque tube : 20 μL d'eau DEPC, 20 μL de tampon 5 X VILO contenant des amorces aléatoires, du $MgCl_2$, des dNTPs et un tampon optimisé pour la RT, 10 μL de SuperScript® Enzyme Mélange réactionnel 10 X contenant la SuperScript® III RT (ADN polymérase ARN dépendante réduisant l'activité des RNaseH) et un inhibiteur des RNase le RNaseOUT™ Recombinant Ribonuclease Inhibitor. Les tubes sont incubés 10 min à température ambiante puis 1 à 3 h à 42°C qui est la température optimale pour que la SuperScript® III RT synthétise l'ADNc. Les brins d'ARN matrice sont détruits en incubant les tubes 5 min à 85°C.

Pour vérifier que la qualité de la RT, une PCR de contrôle est réalisée pour le gène codant pour la β-actine, présent dans toutes cellules non musculaires. Le mélange réactionnel suivant est préparé pour une réaction : 35,6 μL d'eau DEPC (Invitrogen), 5 μL de tampon 10 X (Roche), 1 μL de dNTPs (Amersham Biosciences), 1 μL d'amorce forward, 1 μL d'amorce reverse (Invitrogen), 2 μL de MgCl2 (Roche) et 0,4 μL de Taq (EuroBio). Mélange réactionnel auquel 4 μL de produit de RT sont ajoutés. La PCR est réalisée sur le thermocycler BIO-RAD C1000™ Thermal Cycler selon le programme : 95°C pendant 3 min (94°C pendant 3 sec, 60°C pendant 30 sec, 72°C durant 30 sec) ceci répété 34 fois, 72°C durant 2 min et 12°C ensuite. La migration a est effectuée sur gel d'agarose imprégné de BET, la révélation est faite sous UV.

**5- PCR en temps réel**

La PCR en temps réel est réalisée sur un faible nombre de cycles et en utilisant un fluorochrome dont la fluorescence est proportionnelle à la quantité d'ADN.

Le type de fluorescence détectée ici provient des sondes. L'utilisation de sondes se liant au brin d'ADN correspondant au gène d'intérêt, permet d'obtenir des courbes de florescence proportionnelle à la quantité d'ADN. En effet, une sonde est composée d'ADN complémentaire au gène cible et comprend à une extrémité, un fluorophore et à l'autre extrémité un "quencher" ou fluorophore extincteur. Lorsque les amorces se fixent de manière spécifique au brin d'ADN et que la Taq polymérase progresse le long du brin d'ADN (où est fixée la sonde), le fluorophore est libéré et émet sa fluorescence. Au fur et à mesure des cycles, la fluorescence émise va croitre et sera proportionnelle à la quantité d'amplicons formés.

Les sondes, ont une séquence permettant de détecter des motifs de 8 à 9 nucléotides dont la prévalence dans le transcriptome permet une couverture optimale des gènes. Avec ce type de sonde, la spécificité de la PCR provient des primers. Le cycle-seuil ou Ct (threshold cycle) correspond au cycle au cours duquel la fluorescence de l'ADN amplifié devient significativement différente du bruit de fond. Ce seuil permet de comparer toutes les amplifications pendant la phase exponentielle. Pour que cette relation existe, il est nécessaire que le rendement de l'amplification soit de 100 %, autrement dit, que l'efficacité E du système soit de 1. En effet, si E est différent de 1, la relation entre le Ct et la quantité initiale N0 d'ARN n'est plus linéaire, et toute quantification est alors impossible. L'efficacité E d'une PCR peut être définie en traçant une courbe sur laquelle est représentée le Ct en fonction de la dilution du couple d'amorces. D'après l'équation suivante, la pente de la droite correspond à -1/(log(1+E)). À partir de cette équation on peut écrire E=10-1/pente-1. Donc, lorsque l'efficacité E de la PCR est de 1, la pente de la droite est égale à -1/log2=-3,32. Les couples d'amorces doivent présenter des efficacités E supérieures à 0,85, seuil arbitraire en dessous duquel la PCR n'est plus quantitative. D'autre part, pour comparer la quantité d'ARN présente dans deux échantillons différents, il faut un autre ARN invariant servant de référence c'est-à-dire dont l'efficacité d'amplification soit proche de l'efficacité d'amplification de l'ARN étudié. Ces ARN sont rétro transcrits à partir de gènes dits « gènes de ménages » c'est-à-dire dont la transcription ne varie pas ou peu. Pour comparer les quantités relatives d'ARN d'intérêts entre deux échantillons, il faut dans un premier temps normaliser la quantité d'intérêt relativement à la quantité d'ARN de référence. Cette mesure, appelée $\Delta$Ct correspond au calcul suivant :

$$\Delta Ct = Ct\ ARN - ct\ ARN$$

de référence. Puis on calcule le $\Delta\Delta$Ct = $\Delta$Ctéchantillon1 - $\Delta$Ctéchantillon2 pour obtenir la variation relative exprimée par le nombre $2^{-\Delta\Delta Ct}$.

L'étape de PCR en temps réel est réalisée en utilisant le kit « LightCycler® 480 Probes Master» de Roche.

Pour une réaction, le mélange réactionnel suivant est préparé : 1,4 $\mu$L d'eau « PCR grade », 5 $\mu$L de pré-mélange réactionnel 2 X (contenant l'ADN polymérase Taq Man, le $MgCl_2$, les dNTPs), 0,25 $\mu$L de chaque amorces du gène à quantifier, 0,1 $\mu$L de sonde UPL. L'ADNc est dilué au 1/3 et une gamme étalon de ce dernier est réalisée de 10-1 à 10-3 puis diluée au 1/3 aussi, pour évaluer l'efficacité de la PCR en temps réel. Les mélange réactionnel et ADNc ont été distribués en plaque 384 puits par le biais d'un système de pipetage automatique : epMotion® de vaudau-eppendorf. 7 $\mu$L de mélange réactionnel et 3 $\mu$L d'ADN dilué au 1/3 sont distribués pour une réaction. La PCR en temps réel est effectuée avec le LightCycler® 480 de Roche avec le programme suivant : incubation à 95°C pendant 5 min pour éviter les interactions entre les amorces et donc améliorer la sensibilité, 45 cycles de PCR à 95°C pendant 10 sec et 60°C durant 30 sec et pour finir un refroidissement à 40°C pendant 30 sec. La longueur d'onde d'excitation des sondes est de 465nm et celle de détection de 510nm.

Les résultats obtenus à partir du LightCycler® 480 sont analysés avec le logiciel « LightCycler® 480 Software ».

La qRT-PCR est effectuée sur 7 gènes (isoformes): SOD1, SOD2, CAT, GPX2, GPX1(1), GPX4(1-2-3), PRDX1 (1-2-3) avec calcul des $\Delta$Ct (vs. GAPDH). Le calcul du $\Delta\Delta$Ct ($\Delta$Ct-$\Delta$Ctm) est effectué pour chacun des gènes. Le coefficient de variation par rapport au témoins sains est ensuite déterminé : RQ = 2exp(-$\Delta\Delta$Ct) pour chacun des gènes. Le score S du patient est calculé en utilisant les RQ calculés pour chaque gène, selon la formule suivante :

$$S = \Sigma\ RQ[CAT,SOD1,GPX1(1),GPX4(1\text{-}2\text{-}3),PRDX1(1\text{-}2\text{-}3)] - \Sigma\ RQ[SOD2,GPX2].$$

**Oligonucléotides utilisés**

| gène | Oligo sens | Oligo antisens | sonde |
|---|---|---|---|
| *SOD1* | Gcatcatcaatttcgagcag SEQ ID NO : 34 | Caggccttcagtcagtcctt SEQ ID NO : 35 | tggggaag |
| *SOD2* | Tccactgcaaggaacaacag SEQ ID NO : 42 | Taagcgtgctcccacacat SEQ ID NO : 43 | ctgctggg |
| *CAT* | Cgcagttcggttctccac SEQ ID NO : 32 | Gggtcccgaactgtgtca SEQ ID NO : 33 | tgctggag |
| *GPX1_var1* | Caaccagtttgggcatcag SEQ ID NO : 36 | Gttcacctcgcacttctcg SEQ ID NO : 37 | ggtggtgg |
| *GPX2* | Gtccttggcttcccttgc SEQ ID NO : 44 | Tgttcaggatctcctcattctg SEQ ID NO : 45 | caggagaa |
| *GPX4_var1&2&3* | Tacggacccatggaggag SEQ ID NO : 38 | Ccacacacttgtggagctagaa SEQ ID NO : 39 | ctgcccca |
| *PRDX1_var1&2&3* | Cactgacaaacatggggaagt SEQ ID NO : 40 | Tttgctcttttggacatcagg SEQ ID NO : 41 | ctggctgg |
| *GAPDH* | Agccacatcgctcagacac SEQ ID NO : 46 | Gcccaatacgaccaaatcc SEQ ID NO : 47 | tggggaag |

**Résultats**

L'inventeur est parti de l'hypothèse de départ selon laquelle le mauvais pronostic d'une leucémie myéloïde chronique serait lié à la fréquence en cellules souches leucémiques. Les cellules souches leucémiques présentent un taux réduit de dérivés réactifs de l'oxygène (ou ROS). Aussi, pour maintenir un taux faible de ROS, les cellules souches leucémiques devraient présenter une activité métabolique de détoxification des ROS élevée.

Un des moyens d'augmenter le métabolisme de détoxification des ROS est de modifier le niveau d'expression des gènes codant les enzymes impliquées dans ce processus.

Dans un premier temps, l'inventeur a mesuré par PCR quantitative le niveau d'expression de 25 gènes codant les principales enzymes participant à la détoxification des ROS (et représentés par les séquences SEQ ID NO : 1 à 25, ou les variants de ces séquences lorsqu'ils existent) dans plusieurs échantillons biologiques (qui sont des échantillons sanguins de donneurs non atteints de leucémie, desquels ont été purifiés les polynucléaires neutrophiles) en normalisant ce niveau d'expression à celui de la GAPDH.

Cette mesure du niveau d'expression a permis d'établir un niveau d'expression standard dans les cellules saines.

Dans un second temps, l'inventeur a réalisé, les mêmes types de mesures des niveaux d'expression sur des échantillons lors du diagnostic de 35 patients atteints de leucémie myéloïde chronique par le Service d'Hématologie Biologique du centre hospitalier universitaire de Tours en France.

Les niveaux d'expression de chacun des 25 gènes, pour chacun des 35 patients, ont été comparés au niveau d'expression desdits 25 gènes dans les échantillons sains.

La **figure 1** montre cette comparaison.

On constate de cette figure que l'expression des 25 gènes varie par rapport au niveau d'expression mesuré dans les échantillons sains, avec pour certains gènes une grande variabilité entre les patients. C'est le cas notamment pour les gènes SOD2 : #1, CAT : #7, SOD1 : #8, GPX1(1) : #9, GPX4(1-2-3) : #10, PRDX1(1-2-3) : #12 et GPX2 : #24.

Les 35 patients étant suivis régulièrement sur le long terme, l'inventeur a pu accéder aux données de ceux-ci, notamment leurs réponses moléculaires après un an de traitement avec de l'imatinib mésilate.

Parmi les 35 patients, 10 présentaient une réponse moléculaire majeure à 1 an, 16 présentaient une réponse moléculaire intermédiaire et 9 étaient mauvais répondeurs audit traitement.

En regroupant les patients selon les catégories de réponse susmentionnées, l'inventeur a pu constater que le niveau d'expression desdits 7 gènes susmentionnés variait selon la réponse moléculaire. Les résultats sont présentés à la **figure 2.**

Sur cette base, l'inventeur a proposé pour chaque patient de sommer les valeurs de niveau d'expression pour les gènes dont l'expression était plus élevée dans les mauvais répondeurs, et d'y retrancher le niveau d'expression dont l'expression était plus faible dans les mauvais répondeurs.

Aussi, l'inventeur a proposé la formule

$$S = \Sigma\ RQ[CAT,SOD1,GPX1(1),GPX4(1\text{-}2\text{-}3),PRDX1(1\text{-}2\text{-}3)] - \Sigma\ RQ[SOD2,GPX2].$$

Rétrospectivement, avec les données issues des échantillons de chaque patient prélevés au diagnostic avant le traitement avec l'imatinib mesylate, l'inventeur a pu calculer le score S.

Les données sont regroupées dans les tableaux suivants :

**Patients ayant une réponse moléculaire majeure à 1an**

| patient | SOD2 | CAT | SOD1 | GPX1(1) | GPX4(1-2-3) | PRDX1(1-2-3) | GPX2 | Score S |
|---|---|---|---|---|---|---|---|---|
| **1** | 0,04 | 0,16 | 0,29 | 0,15 | 0,16 | 0,32 | 0,00 | **1,03** |
| **2** | 0,05 | 0,07 | 0,14 | 0,10 | 0,08 | 0,13 | 0,13 | **0,33** |
| **3** | 0,06 | 0,14 | 0,26 | 0,09 | 0,22 | 0,16 | 0,73 | **0,09** |
| **4** | 0,01 | 0,30 | 0,41 | 0,26 | 0,29 | 0,59 | 0,01 | **1,83** |
| **5** | 0,02 | 0,06 | 0,08 | 0,07 | 0,13 | 0,04 | 0,10 | **0,27** |
| **6** | 0,01 | 0,32 | 0,26 | 0,26 | 0,23 | 0,38 | 0,02 | **1,42** |
| **7** | 0,01 | 0,43 | 0,36 | 0,79 | 0,62 | 0,53 | 0,04 | **2,68** |
| **8** | 0,16 | 0,19 | 0,37 | 0,17 | 0,28 | 0,49 | 0,42 | **0,93** |
| **9** | 0,01 | 0,18 | 0,30 | 0,14 | 0,06 | 0,32 | 0,01 | **0,99** |
| **10** | 0,02 | 0,20 | 0,33 | 0,09 | 0,06 | 0,54 | 0,04 | **1,16** |

**Patients ayant une réponse moléculaire intermédiaire à 1 an**

| patient | SOD2 | CAT | SOD1 | GPX1(1) | GPX4(1-2-3) | PRDX1(1-2-3) | GPX2 | score S |
|---|---|---|---|---|---|---|---|---|
| **11** | 0,01 | 0,29 | 0,24 | 0,09 | 0,23 | 0,26 | 0,00 | **1,10** |
| **12** | 0,00 | 0,10 | 0,17 | 0,02 | 0,05 | 0,07 | 0,00 | **0,40** |
| **13** | 0,02 | 0,08 | 0,25 | 0,22 | 0,18 | 0,38 | 0,00 | **1,09** |
| **14** | 0,01 | 0,29 | 0,48 | 0,29 | 0,35 | 0,63 | 0,03 | **2,01** |
| **15** | 0,02 | 0,34 | 0,18 | 0,05 | 0,07 | 0,13 | 0,01 | **0,74** |
| **16** | 0,01 | 0,24 | 0,21 | 0,10 | 0,16 | 0,10 | 0,06 | **0,75** |
| **17** | 0,01 | 0,49 | 0,85 | 0,31 | 0,66 | 0,69 | 0,01 | **2,98** |
| **18** | 0,01 | 0,15 | 0,34 | 0,10 | 0,30 | 0,34 | 0,00 | **1,21** |
| **19** | 0,01 | 0,74 | 0,57 | 1,20 | 1,15 | 1,49 | 0,09 | **5,06** |
| **20** | 0,08 | 0,12 | 0,22 | 0,08 | 0,16 | 0,08 | 0,00 | **0,57** |
| **21** | 0,01 | 0,35 | 0,29 | 0,48 | 0,36 | 0,98 | 0,02 | **2,43** |
| **22** | 0,01 | 0,09 | 0,07 | 0,04 | 0,03 | 0,12 | 0,06 | **0,27** |
| **23** | 0,02 | 0,11 | 0,45 | 0,75 | 0,24 | 0,44 | 0,00 | **1,97** |
| **24** | 0,02 | 0,19 | 0,16 | 0,06 | 0,20 | 0,28 | 0,00 | **0,87** |
| **25** | 0,00 | 0,37 | 0,33 | 0,13 | 0,27 | 0,29 | 0,00 | **1,38** |
| **26** | 0,04 | 0,18 | 0,17 | 0,12 | 0,12 | 0,16 | 0,01 | **0,70** |

**Patients mauvais répondeurs à 1 an**

| patient | SOD2 | CAT | SOD1 | GPX1(1) | GPX4(1-2-3) | PRDX1(1-2-3) | GPX2 | score S |
|---|---|---|---|---|---|---|---|---|
| **27** | 0,02 | 0,16 | 0,93 | 1,50 | 0,55 | 2,88 | 0,01 | **5,99** |
| **28** | 0,01 | 0,34 | 0,26 | 0,19 | 0,11 | 0,38 | 0,03 | **1,24** |
| **29** | 0,01 | 0,38 | 1,00 | 0,29 | 0,44 | 1,15 | 0,01 | **3,25** |
| **30** | 0,01 | 0,53 | 0,80 | 0,24 | 0,31 | 0,48 | 0,00 | **2,34** |
| **31** | 0,00 | 0,19 | 0,47 | 0,27 | 0,38 | 0,41 | 0,00 | **1,70** |
| **32** | 0,03 | 0,51 | 0,67 | 0,38 | 0,56 | 0,92 | 0,01 | **3,00** |
| **33** | 0,07 | 0,12 | 0,32 | 0,50 | 0,30 | 0,15 | 0,01 | **1,30** |
| **34** | 0,02 | 0,22 | 0,53 | 1,11 | 0,42 | 0,71 | 0,00 | **2,97** |
| **35** | 0,01 | 0,15 | 0,31 | 0,12 | 0,18 | 0,35 | 0,00 | **1,10** |

En reclassant les patients selon le score S, l'inventeur a pu constater de manière surprenante que :

si S<1, environ 40% des patients auront une réponse moléculaire majeure à 1 an, et environ 60 % des patients auront une réponse moléculaire intermédiaire à 1 an. En d'autres termes, 100% des patients auront une réponse moléculaire, et aucun ne sera mauvais répondeur,

si 1<S<2, environ 30 % des patients auront un réponse moléculaire majeure à 1an, environ 40% des patient auront une réponse moléculaire intermédiaire à 1 an, et surtout environ 30% des patients seront de mauvais répondeurs. En d'autres termes, environ 70% des patients présenterons une réponse moléculaire, et

si S>2, seuls 10% des patients auront une réponse moléculaire majeure, environ 30% des patients auront une réponse moléculaire intermédiaire, et plus de 50 % des patients seront des mauvais répondeurs. En d'autres termes, seuls environ 40% des patients répondront au traitement à l'imatinib.

Les résultats obtenus pour les 35 patients sont présentés dans le tableau suivant :

| | n = 10 | n = 16 | n = 26 | n = 9 |
|---|---|---|---|---|
| score | RMM | RMI | R | MR |
| S < 1 | 41,7% | 58,3% | 100,0% | 0,0% |
| 1 < S < 2 | 28,6% | 42,9% | 71,4% | 28,6% |
| S > 2 | 11,1% | 33,3% | 44,4% | 55,6% |
| RMM : réponse moléculaire majeure, RMI : réponse moléculaire intermédiaire, R : réponse, MR : mauvais répondeur. | | | | |

La **figure 3** est une représentation graphique de ces résultats, présentant la répartition des réponses moléculaires à un an (noir: majeure ; hachuré : intermédiaire ; gris : non répondeur) en fonction du score S.

La **figure 4** quant à elle montre la distribution des patients selon leur score S.

L'invention n'est pas limitée aux modes de réalisation présentés et d'autres modes de réalisation apparaîtront clairement à l'homme du métier.

**Revendications**

**1.** Méthode de pronostic *in vitro* de la réponse à une thérapie d'un individu atteint d'une leucémie myéloïde chronique, à partir d'un échantillon biologique leucémique issu dudit individu, ladite méthode comprenant :

a. une étape de mesure du niveau d'expression des gènes d'au moins un sous-groupe de gènes choisis dans un groupe de gènes,
ledit groupe de gènes étant constitué de 25 gènes, lesdits 25 gènes comprenant ou étant constitués par les séquences d'acides nucléiques SEQ ID NO : 1 à SEQ ID NO : 25,
ledit sous-groupe consistant en 7 gènes, lesdits 7 gènes comprenant ou étant constitués par les séquences d'acides nucléiques SEQ ID NO : 1 à SEQ ID NO: 7,
une valeur du niveau d'expression mesuré étant obtenue pour chacun des gènes dudit sous-groupe,
b. une étape de comparaison de la valeur attribuée à l'étape précédente à chacun desdits gènes dudit sous-groupe à la valeur attribuée à chacun desdits gènes dudit sous-groupe obtenue à partir d'un échantillon biologique sain, afin d'obtenir un ratio pour chacun desdits gènes dudit sous-groupe du niveau d'expression dans l'échantillon biologique leucémique sur le niveau d'expression dans l'échantillon sain, et
c. une étape de détermination d'un score S selon la formule suivante

$$S = \sum ratio\ i - \sum ratio\ j$$

où ratio i et ratio j représentent respectivement les ratios obtenus pour lesdits gènes dudit sous-groupe comprenant ou étant constitués par les séquences d'acides nucléiques SEQ ID NO : i ou SEQ ID NO : j, où i et j sont des entiers, i variant de 1 à 5 et j variant de 6 à 7,
de sorte que :

- si S est inférieur à 1, ledit individu aura au moins environ 40% de chances de présenter une rémission moléculaire majeure un an après le début d'une thérapie avec un inhibiteur de tyrosine kinase de première génération, et

- si S est supérieur ou égal à 1, ledit individu aura moins d'environ 40% de chances de présenter une rémission moléculaire majeure un an après le début d'une thérapie avec un inhibiteur de tyrosine kinase de première génération.

**2.** Méthode de pronostic *in vitro* selon la revendication 1, dans laquelle

- si S est supérieur ou égal à 1 et inférieur ou égal à 2, ledit individu aura de 40% à 10% de chances de présenter une rémission moléculaire majeure un an après le début d'une thérapie avec un inhibiteur de tyrosine kinase de première génération.

**3.** Méthode de pronostic *in vitro* selon la revendication 1 ou 2, dans laquelle

- si S est supérieur à 2, ledit individu aura moins de 10% de chances de présenter une rémission moléculaire un an après le début d'une thérapie avec un inhibiteur de tyrosine kinase de première génération.

**4.** Méthode de pronostic *in vitro* selon l'une quelconque des revendications 1 à 3, dans laquelle la valeur du niveau d'expression mesuré est obtenue par une mesure de l'expression desdits gènes du sous-groupe par une méthode de mesure quantitative, notamment la méthode de PCR quantitative.

**5.** Méthode de pronostic *in vitro* selon l'une quelconque des revendications 1 à 4, dans laquelle la valeur du niveau d'expression mesuré est obtenue par une mesure de l'expression desdits gènes du sous-groupe mise en œuvre en utilisant au moins les oligonucléotides comprenant ou constitués des séquences SEQ ID :32 à 45.

**6.** Méthode de pronostic *in vitro* selon l'une quelconque des revendications 1 à 5, où la valeur du niveau d'expression mesuré est obtenue par une mesure de l'expression desdits gènes du sous-groupe, ladite mesure utilisant les oligonucléotides suivants :

- les oligonucléotides SEQ ID NO : 32 et 33 pour mesurer l'expression du gène comprenant ou étant constitué par la séquence d'acides nucléiques SEQ ID NO : 1,
- les oligonucléotides SEQ ID NO : 34 et 35 pour mesurer l'expression du gène comprenant ou étant constitué par la séquence d'acides nucléiques SEQ ID NO : 2
- les oligonucléotides SEQ ID NO : 36 et 37 pour mesurer l'expression du gène comprenant ou étant constitué par la séquence d'acides nucléiques SEQ ID NO : 3
- les oligonucléotides SEQ ID NO : 38 et 39 pour mesurer l'expression du gène comprenant ou étant constitué par la séquence d'acides nucléiques SEQ ID NO : 4
- les oligonucléotides SEQ ID NO : 40 et 41 pour mesurer l'expression du gène comprenant ou étant constitué par la séquence d'acides nucléiques SEQ ID NO : 5
- les oligonucléotides SEQ ID NO : 42 et 43 pour mesurer l'expression du gène comprenant ou étant constitué par la séquence d'acides nucléiques SEQ ID NO : 6, et
- les oligonucléotides SEQ ID NO : 44 et 45 pour mesurer l'expression du gène comprenant ou étant constitué par la séquence d'acides nucléiques SEQ ID NO : 7.

**7.** Méthode de théranostic *in vitro,* d'un individu atteint d'une leucémie myéloïde chronique, comprenant

a. une étape de mesure du niveau d'expression d'au moins un sous-groupe de gènes choisis dans un groupe de gènes,
ledit groupe de gènes étant constitué de 25 gènes, lesdits 25 gènes comprenant ou étant constitués par les séquences d'acides nucléiques SEQ ID NO : 1 à SEQ ID NO: 25,
ledit sous-groupe consistant en 7 gènes, lesdits 7 gènes comprenant ou étant constitués par les séquences d'acides nucléiques SEQ ID NO : 1 à SEQ ID NO: 7,
une valeur du niveau d'expression mesuré étant obtenue pour chacun des gènes dudit sous-groupe,
b. une étape de comparaison de la valeur attribuée à l'étape précédente à chacun desdits gènes dudit sous-groupe à la valeur attribuée à chacun desdits gènes dudit sous-groupe obtenue à partir d'un échantillon biologique sain, afin d'obtenir un ratio pour chacun desdits gènes dudit sous-groupe du niveau d'expression dans l'échantillon biologique leucémique sur le niveau d'expression dans l'échantillon sain, et
c. une étape de détermination d'un score S selon la formule suivante

$$S = \sum ratio\ i - \sum ratio\ j$$

où ratio i et ratio j représentent respectivement les ratios obtenus pour lesdits gènes dudit sous-groupe comprenant ou étant constitués par les séquences d'acides nucléiques SEQ ID NO : i ou SEQ ID NO : j, où i et j sont des entiers, i variant de 1 à 5 et j variant de 6 à 7,
de sorte que

- si S est inférieur à 1, la leucémie myéloïde chronique dudit individu est une leucémie myéloïde chronique susceptible de répondre préférentiellement à un traitement comprenant un inhibiteur de tyrosine kinase de première génération, et
- si S est supérieur à 2, la leucémie myéloïde chronique dudit individu est une leucémie myéloïde chronique susceptible de répondre préférentiellement à un traitement comprenant un inhibiteur de tyrosine kinase de seconde génération.

**8.** Méthode selon l'une quelconque des revendications 1 à 7, dans laquelle l'inhibiteur de tyrosine kinase de première génération est l'imatinib ou l'un de ses sels.

**9.** Méthode selon la revendication 7, dans laquelle l'inhibiteur de tyrosine kinase de seconde génération est le dasatinib ou le nilotinib, ou l'un de leurs sels.

**10.** Produit programme d'ordinateur compris dans un support approprié conçu pour la mise en œuvre des étapes b et c de la méthode de pronostic telle que définie dans l'une quelconque des revendications 1 à 9, comprenant des instructions de code de programme pour l'exécution de ladite méthode de pronostic lorsque ledit programme est exécuté sur un ordinateur.

**Patentansprüche**

**1.** Verfahren zur In-vitro-Prognose des Ansprechens eines an chronischer myeloischer Leukämie erkrankten Lebewesens auf eine Therapie, ausgehend von einer leukämischen biologischen Probe, die von diesem Lebewesen stammt, wobei das Verfahren Folgendes umfasst:

a. einen Schritt zum Messen des Expressionsniveaus der Gene von wenigstens Untergruppe von Genen, die aus einer Gruppe von Genen ausgewählt sind,
wobei die Gruppe von Genen aus 25 Genen besteht, wobei die 25 Gene die Nukleinsäure-Sequenzen SEQ ID Nr. 1 bis SEQ ID Nr. 25 enthalten oder daraus bestehen,
wobei die Untergruppe aus 7 Genen besteht, wobei die 7 Gene die Nukleinsäure-Sequenzen SEQ ID Nr. 1 bis SEQ ID Nr. 7 enthalten oder daraus bestehen,
wobei ein Wert des gemessenen Expressionsniveaus für jedes der Gene der Untergruppe erhalten wird,
b. einen Schritt des Vergleichens des Wertes, der im vorhergehenden Schritt jedem der Gene der Untergruppe zugewiesen wurde, mit dem Wert, der jedem der Gene der Untergruppe zugewiesen wurde und ausgehend von einer gesunden biologischen Probe erhalten wurde, um ein Verhältnis für jedes der Gene der Untergruppe des Expressionsniveaus in der leukämischen biologischen Probe gegenüber dem Expressionsniveau in der gesunden Probe zu erhalten, und
c. einen Schritt des Bestimmens einer Bewertungsziffer S gemäß der folgenden Formel:

$$S = \sum Verhältnis\ i - \sum Verhältnis\ j$$

worin Verhältnis i und Verhältnis j jeweils die Verhältnisse darstellen, die für die Gene der Untergruppe erhalten wurden, welche die Nukleinsäure-Sequenzen SEQ ID Nr. i oder SEQ ID Nr. j enthalten oder daraus bestehen, worin i und j ganze Zahlen sind, wobei i von 1 bis 5 variiert und j von 6 bis 7 variiert, so dass:

- wenn S kleiner als 1 ist, das Lebewesen zu wenigstens etwa 40 % Aussichten auf eine majore molekulare Remission ein Jahr nach dem Beginn einer Therapie mit einem Tyrosinkinaseinhibitor der ersten Generation hat, und
- wenn S größer oder gleich 1 ist, das Lebewesen zu weniger als etwa 40 % Aussichten auf eine majore molekulare Remission ein Jahr nach dem Beginn einer Therapie mit einem Tyrosinkinaseinhibitor der ersten Generation hat.

**2.** Verfahren zur In-vitro-Prognose nach Anspruch 1, bei dem

- wenn S größer oder gleich 1 und kleiner oder gleich 2 ist, das Lebewesen zu 40 % bis 10 % Aussichten auf eine majore molekulare Remission ein Jahr nach dem Beginn einer Therapie mit einem Tyrosinkinaseinhibitor der ersten Generation hat.

**3.** Verfahren zur In-vitro-Prognose nach Anspruch 1 oder 2, bei dem

- wenn S größer als 2 ist, das Lebewesen zu weniger als 10 % Aussichten auf eine molekulare Remission ein Jahr nach dem Beginn einer Therapie mit einem Tyrosinkinaseinhibitor der ersten Generation hat.

**4.** Verfahren zur In-vitro-Prognose nach einem der Ansprüche 1 bis 3, bei dem der Wert des gemessenen Expressionsniveaus durch eine Messung der Expression der Gene der Untergruppe durch ein quantitatives Messverfahren erhalten wird, insbesondere das quantitative PCR-Verfahren.

**5.** Verfahren zur In-vitro-Prognose nach einem der Ansprüche 1 bis 4, bei dem der Wert des gemessenen Expressionsniveaus durch ein Verfahren zur Messung der Expression der Gene der Untergruppe erhalten wird, das unter Verwendung wenigstens der Oligonukleotide, welche die Sequenzen SEQ ID Nr. 32 bis 45 enthalten oder daraus bestehen, ausgeführt wird.

**6.** Verfahren zur *In-vitro*-Prognose nach einem der Ansprüche 1 bis 5, bei dem der Wert des gemessenen Expressionsniveaus durch eine Messung der Expression der Gene der Untergruppe erhalten wird, wobei die Messung die folgenden Oligonukleotide verwendet:

- die Oligonukleotide SEQ ID Nr: 32 und 33, um die Expression des Gens zu messen, das die Nukleinsäure-Sequenz SEQ ID Nr. 1 enthält oder daraus besteht,
- die Oligonukleotide SEQ ID Nr: 34 und 35, um die Expression des Gens zu messen, das die Nukleinsäure-Sequenz SEQ ID Nr. 2 enthält oder daraus besteht,
- die Oligonukleotide SEQ ID Nr: 36 und 37, um die Expression des Gens zu messen, das die Nukleinsäure-Sequenz SEQ ID Nr. 3 enthält oder daraus besteht,
- die Oligonukleotide SEQ ID Nr: 38 und 39, um die Expression des Gens zu messen, das die Nukleinsäure-Sequenz SEQ ID Nr. 4 enthält oder daraus besteht,
- die Oligonukleotide SEQ ID Nr: 40 und 41, um die Expression des Gens zu messen, das die Nukleinsäure-Sequenz SEQ ID Nr. 5 enthält oder daraus besteht,
- die Oligonukleotide SEQ ID Nr: 42 und 43, um die Expression des Gens zu messen, das die Nukleinsäure-Sequenz SEQ ID Nr. 6 enthält oder daraus besteht, und
- die Oligonukleotide SEQ ID Nr: 44 und 45, um die Expression des Gens zu messen, das die Nukleinsäure-Sequenz SEQ ID Nr. 7 enthält oder daraus besteht.

**7.** Verfahren zur In-Vitro-Theranose, eines an chronischer myeloischer Leukämie erkrankten Lebewesens, umfassend

a. einen Schritt zum Messen des Expressionsniveaus wenigstens einer Untergruppe von Genen, die aus einer Gruppe von Genen ausgewählt sind,
wobei die Gruppe von Genen aus 25 Genen besteht, wobei die 25 Gene die Nukleinsäure-Sequenzen SEQ ID Nr. 1 bis SEQ ID Nr. 25 enthalten oder daraus bestehen,
wobei die Untergruppe aus 7 Genen besteht, wobei die 7 Gene die Nukleinsäure-Sequenzen SEQ ID Nr. 1 bis SEQ ID Nr. 7 enthalten oder daraus bestehen,
wobei ein Wert des gemessenen Expressionsniveaus für jedes der Gene der Untergruppe erhalten wird,
b. einen Schritt des Vergleichens des Wertes, der im vorhergehenden Schritt jedem der Gene der Untergruppe zugewiesen wurde, mit dem Wert, der jedem der Gene der Untergruppe zugewiesen wurde und ausgehend von einer gesunden biologischen Probe erhalten wurde, um ein Verhältnis für jedes der Gene der Untergruppe des Expressionsniveaus in der leukämischen biologischen Probe gegenüber dem Expressionsniveau in der gesunden Probe zu erhalten, und

c. einen Schritt des Bestimmens einer Bewertungsziffer S gemäß der folgenden Formel:

$$S = \sum Verhältnis\ i - \sum Verhältnis\ j$$

worin Verhältnis i und Verhältnis j jeweils die Verhältnisse darstellen, die für die Gene der Untergruppe erhalten wurden, welche die Nukleinsäure-Sequenzen SEQ ID Nr. i oder SEQ ID Nr. j enthalten oder daraus bestehen,
worin i und j ganze Zahlen sind, wobei i von 1 bis 5 variiert und j von 6 bis 7 variiert, so dass:

- wenn S kleiner als 1 ist, die chronische myeloische Leukämie des Lebewesens eine chronische myeloische Leukämie ist, die in der Lage ist, bevorzugt auf eine Behandlung anzusprechen, die einen Tyrosinkinaseinhibitor der ersten Generation enthält, und
- wenn S größer als 2 ist, die chronische myeloische Leukämie des Lebewesens eine chronische myeloische Leukämie ist, die in der Lage ist, bevorzugt auf eine Behandlung anzusprechen, die einen Tyrosinkinaseinhibitor der zweiten Generation enthält.

**8.** Verfahren nach einem der Ansprüche 1 bis 7, bei dem der Tyrosinkinaseinhibitor der ersten Generation Imatinib oder eines der Salze davon ist.

**9.** Verfahren nach Anspruch 7, bei dem der Tyrosinkinaseinhibitor der zweiten Generation Dasatinib oder Nilotinib oder eines der Salze davon ist.

**10.** Computerprogrammprodukt, das in einem geeigneten Träger enthalten ist und ausgelegt ist für die Ausführung der Schritte b und c des Prognoseverfahrens nach einem der Ansprüche 1 bis 9, umfassend Programmcodeanweisungen zum Ausführen des Prognoseverfahrens, wenn das Programm auf einem Computer ausgeführt wird.

## Claims

**1.** An *in vitro* method of prognosis for the response to a treatment of an individual suffering from chronic myeloid leukemia, based on a leukemic biological sample taken from said individual, said method comprising:

a. a step of measuring the expression level of the genes from at least one subgroup of genes chosen from a group of genes,
said group of genes consisting of 25 genes, said 25 genes comprising or consisting of the nucleic acid sequences as set forth in SEQ ID NO: 1 to SEQ ID NO: 25,
said subgroup consisting in 7 genes, said 7 genes comprising or consisting of the nucleic acid sequences as set forth in SEQ ID NO: 1 to SEQ ID NO: 7,
a value of the measured expression level being obtained for each of the genes of said subgroup,
b. a step of comparing the value attributed to the preceding step with each of said genes of said subgroup to the value attributed to each of said genes of said subgroup obtained from a healthy biological sample, in order to obtain a ratio for each of said genes of said subgroup of the expression level in the leukemic biological sample to the expression level in the healthy sample, and
c. a step of determining a score S according to the following formula

$$S = \sum ratio\ i - \sum ratio\ j$$

where ratio i and ratio j represent respectively the ratios obtained for said genes of said subgroup comprising or consisting of the nucleic acid sequences as set forth in SEQ ID NO: i or SEQ ID NO: j,
where i and j are integers, i varying from 1 to 5 and j varying from 6 to 7, so that:

- if S is less than 1, said individual will have more than around a 40% chance of having a major molecular remission one year after the start of treatment with a first-generation tyrosine kinase inhibitor, and
- if S is greater than or equal to 1, said individual will have less than around a 40% chance of having a major molecular remission one year after the start of treatment with a first-generation tyrosine kinase inhibitor.

**2.** The in *vitro* method of prognosis according to claim 1, wherein

- if S is greater than or equal to 1 and less than or equal to 2, said individual will have from a 40% to a 10% chance of having a major molecular remission one year after the start of treatment with a first-generation tyrosine kinase inhibitor.

**3.** The *In vitro* method of prognosis according to claim 1, wherein

- if S is greater than 2, said individual will have less than a 10% chance of having a major molecular remission one year after starting treatment with a first-generation tyrosine kinase inhibitor.

**4.** The in *vitro* method of prognosis according to anyone of claims 1 to 3, wherein the value of the expression level measured is obtained by a measurement of the expression of said genes of the subgroup by using a quantitative measuring method, in particular the quantitative PCR method.

**5.** *The in vitro* method of prognosis according to anyone of claims 1 to 4, wherein the value of the measured expression level is obtained by a measurement of the expression of said genes of the subgroup implemented by using at least those oligonucleotides comprising or consisting of sequences as set forth in SEQ ID: 32 to 45.

**6.** The *in vitro* method of prognosis according to anyone of claims 1 to 5, wherein the value of the measured expression level is obtained by a measurement of the expression of said genes of the subgroup, said measurement using the following oligonucleotides:

- oligonucleotides SEQ ID NOS: 32 and 33 to measure the expression of the gene comprising or consisting of the sequence of nucleic acids as set forth in SEQ ID NO: 1,
- oligonucleotides SEQ ID NOS: 34 and 35 to measure the expression of the gene comprising or or consisting of the sequence of nucleic acids as set forth in SEQ ID NO: 2,
- oligonucleotides SEQ ID NOS: 36 and 37 to measure the expression of the gene comprising or consisting of the sequence of nucleic acids as set forth in SEQ ID NO: 3,
- oligonucleotides SEQ ID NOS: 38 and 39 to measure the expression of the gene comprising consisting of the sequence of nucleic acids as set forth in SEQ ID NO: 4,
- oligonucleotides SEQ ID NOS: 40 and 41 to measure the expression of the gene comprising consisting of the sequence of nucleic acids as set forth in SEQ ID NO: 5,
- oligonucleotides SEQ ID NOS: 42 and 43 to measure the expression of the gene comprising consisting of the sequence of nucleic acids as set forth in SEQ ID NO: 6, and
- oligonucleotides SEQ ID NOS: 44 and 45 to measure the expression of the gene comprising consisting of the sequence of nucleic acids as set forth in SEQ ID NO: 7.

**7.** An *in vitro* theranostic method of an individual suffering from chronic myeloid leukemia, comprising:

a. a step of measuring the expression level of the genes from at least one subgroup of genes chosen from a group of genes,
said group of genes consisting of 25 genes, said 25 genes comprising or consisting of the nucleic acid sequences as set forth in SEQ ID NO: 1 to SEQ ID NO: 25,
said subgroup consisting in 7 genes, said 7 genes comprising or consisting of the nucleic acid sequences as set forth in SEQ ID NO: 1 to SEQ ID NO: 7,
a value of the expression level measured being obtained for each of the genes of said subgroup,
b. a step of comparing the value attributed to the preceding step with each of said genes of said subgroup to the value attributed to each of said genes of said subgroup obtained from a healthy biological sample, in order to obtain a ratio for each of said genes of said subgroup of the expression level in the leukemic biological sample to the expression level in the healthy sample, and
c. a step of determining a score S according to the following formula

$$S = \sum ratio\ i - \sum ratio\ j$$

where ratio i and ratio j represent respectively the ratios obtained for said genes of said subgroup comprising or consisting of the nucleic acid sequences as set forth in SEQ ID NO: i or SEQ ID NO: j,
where i and j are integers, i varying from 1 to 5 and j varying from 6 to 7, so that:

- if S is less than 1, the chronic myeloid leukemia of said individual is chronic myeloid leukemia likely to respond preferentially to a treatment comprising a first-generation tyrosine kinase inhibitor, and
- if S is greater than 2, the chronic myeloid leukemia of said individual is chronic myeloid leukemia likely to respond preferentially to a treatment comprising a second-generation tyrosine kinase inhibitor.

**8.** The Method according to any of claims 1 to 7, wherein the first-generation tyrosine kinase inhibitor is imatinib or one of its salts.

**9.** Method according to claim 7, wherein the second-generation tyrosine kinase inhibitor is dasatinib or nilotinib, or one of their salts.

**10.** A computer program product comprised in an appropriate support designed to implement step b and c of the method of prognosis as defined in any of claims 1 to 9, comprising instructions of program code for executing said method of prognosis when said program is executed on a computer.

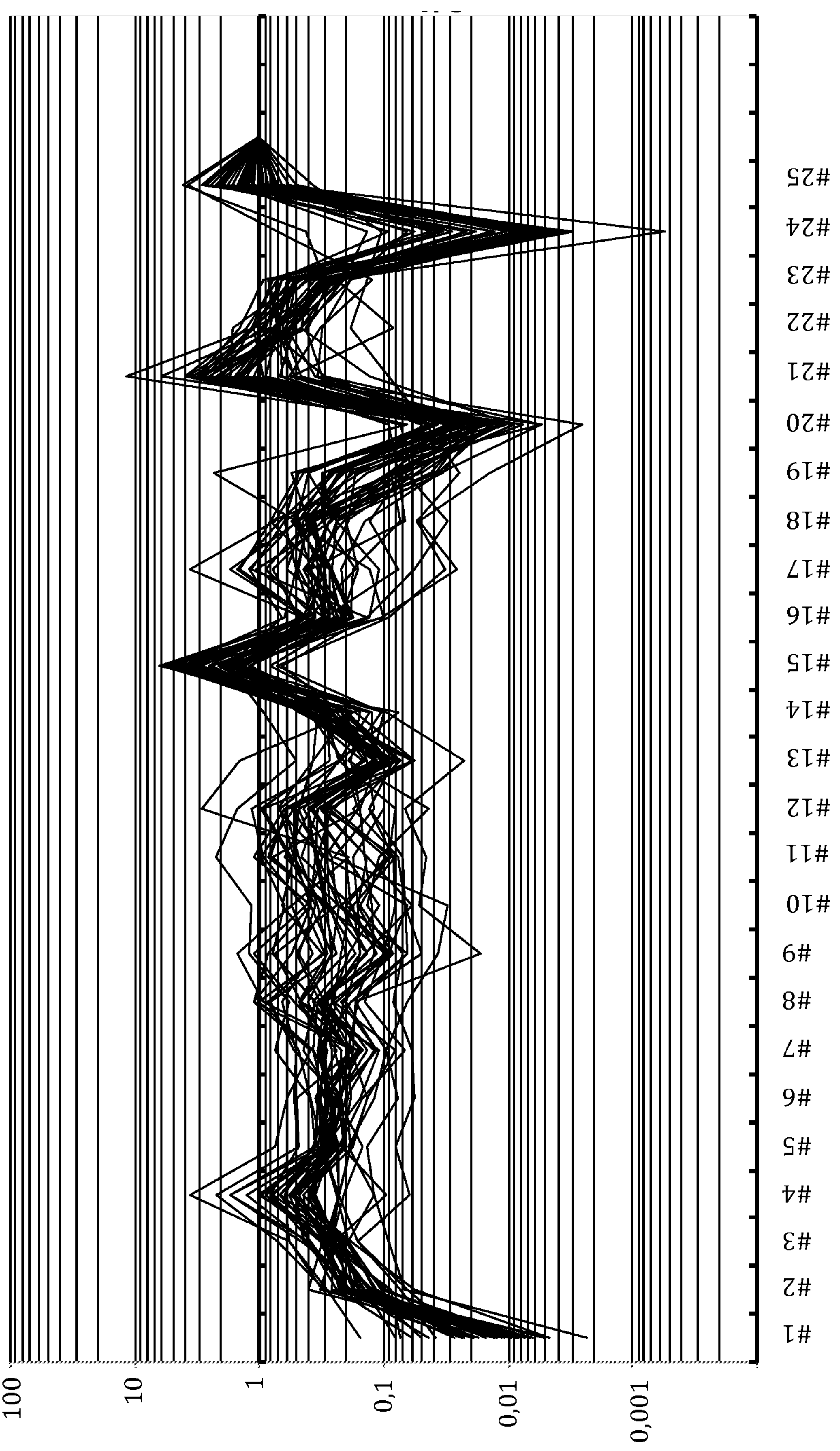
100
10
1
0,1
0,01
0,001
#1
#2
#3
#4
#5
#6
#7
#8
#9
#10
#11
#12
#13
#14
#15
#16
#17
#18
#19
#20
#21
#22
#23
#24
#25

Figure 1

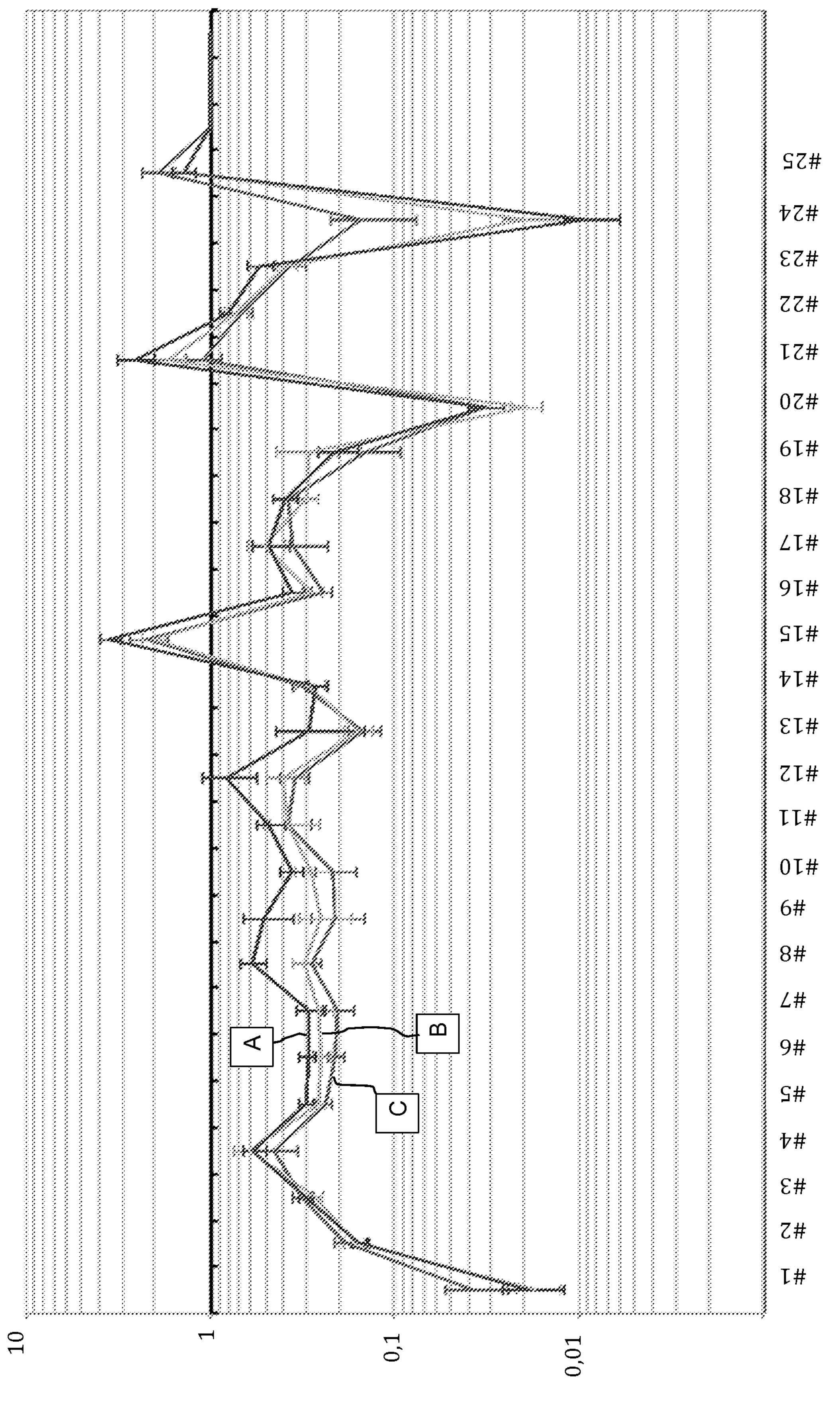
10
1
0,1
0,01
#1
#2
#3
#4
#5
#6
#7
#8
#9
#10
#11
#12
#13
#14
#15
#16
#17
#18
#19
#20
#21
#22
#23
#24
#25
A
B
C

**Figure 2**

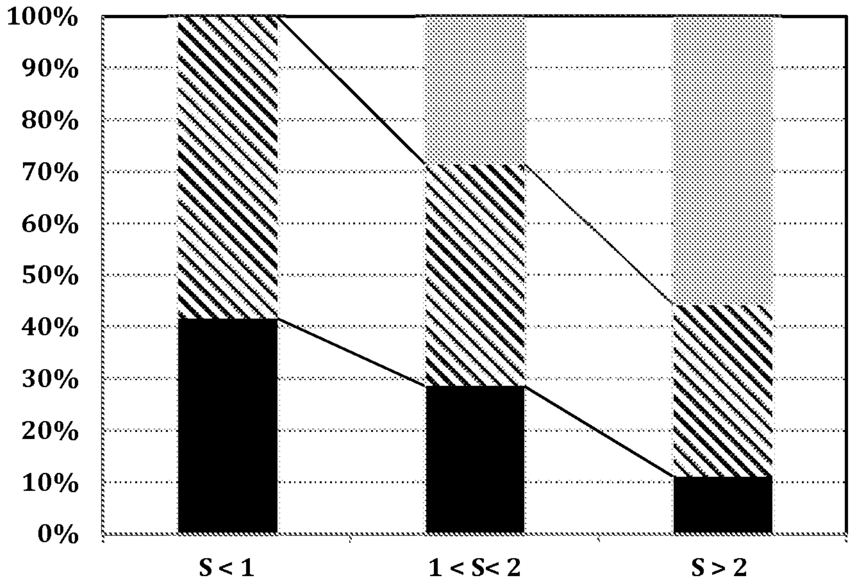


**Figure 3**

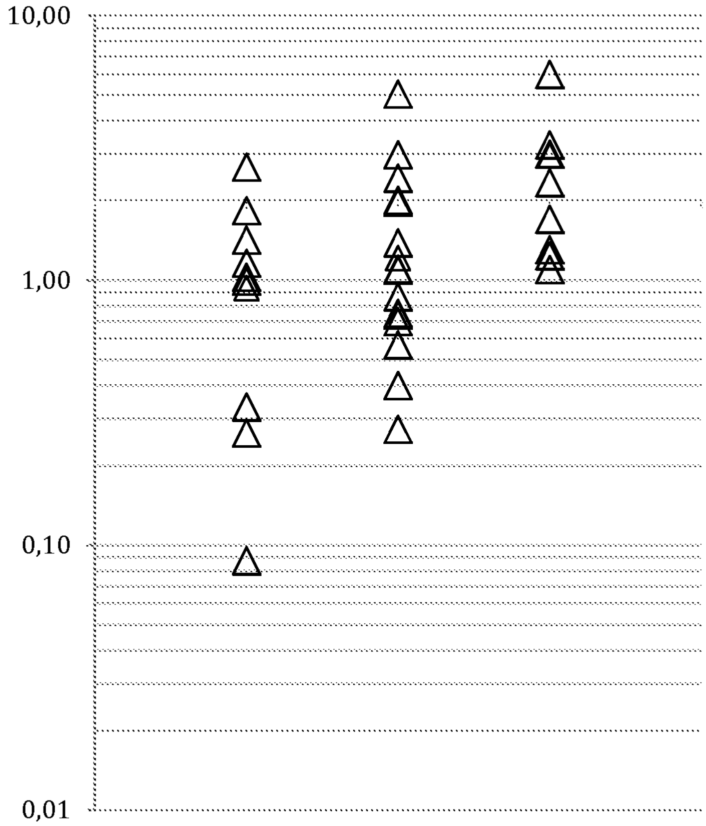


**Figure 4**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2012049329 A
- BR 76137
- FR 1461553


### Littérature non-brevet citée dans la description


- **BACCARANI et al.** *Blood,* 2013, vol. 122 (6), 872-884

SEQUENCE LISTING

<110> CENTRE NATIONAL DE LA RECHERCHE SCIENTIFIQUE (CNRS) UNIVERSITE FRANCOIS RABELAIS DE TOURS

<120> Methode de pronostic d'hemopathies

<130> BR 76137

<150> FR 14/61553
<151> 2014-11-27

<160> 47

<170> PatentIn version 3.5

<210> 1
<211> 2300
<212> DNA
<213> Homo sapiens

<400> 1

actcggggca acaggcagat ttgcctgctg agggtggaga cccacgagcc gaggcctcct 60
gcagtgttct gcacagcaaa ccgcacgcta tggctgacag ccgggatccc gccagcgacc 120
agatgcagca ctggaaggag cagcgggccg cgcagaaagc tgatgtcctg accactggag 180
ctggtaaccc agtaggagac aaacttaatg ttattacagt agggccccgt gggccccttc 240
ttgttcagga tgtggttttc actgatgaaa tggctcattt tgaccgagag agaattcctg 300
agagagttgt gcatgctaaa ggagcagggg cctttggcta ctttgaggtc acacatgaca 360
ttaccaaata ctccaaggca aaggtatttg agcatattgg aaagaagact cccatcgcag 420
ttcggttctc cactgttgct ggagaatcgg gttcagctga cacagttcgg gaccctcgtg 480
ggtttgcagt gaaattttac acagaagatg gtaactggga tctcgttgga aataacaccc 540
ccatttcttt catcagggat cccatattgt ttccatcttt tatccacagc caaaagagaa 600
atcctcagac acatctgaag gatccggaca tggtctggga cttctggagc ctacgtcctg 660
agtctctgca tcaggtttct ttcttgttca gtgatcgggg gattccagat ggacatcgcc 720
acatgaatgg atatggatca catactttca agctggttaa tgcaaatggg gaggcagttt 780
attgcaaatt ccattataag actgaccagg gcatcaaaaa cctttctgtt gaagatgcgg 840
cgagactttc ccaggaagat cctgactatg gcatccggga tctttttaac gccattgcca 900
caggaaagta cccctcctgg actttttaca tccaggtcat gacatttaat caggcagaaa 960
cttttccatt taatccattc gatctcacca aggtttggcc tcacaaggac taccctctca 1020
tcccagttgg taaactggtc ttaaaccgga atccagttaa ttactttgct gaggttgaac 1080
agatagcctt cgacccaagc aacatgccac ctggcattga ggccagtcct gacaaaatgc 1140
ttcagggccg cctttttgcc tatcctgaca ctcaccgcca tcgcctggga cccaattatc 1200
ttcatatacc tgtgaactgt ccctaccgtg ctcgagtggc caactaccag cgtgacggcc 1260

cgatgtgcat gcaggacaat cagggtggtg ctccaaatta ctaccccaac agctttggtg 1320

ctccggaaca acagccttct gccctggagc acagcatcca atattctgga gaagtgcgga 1380

gattcaacac tgccaatgat gataacgtta ctcaggtgcg ggcattctat gtgaacgtgc 1440

tgaatgagga acagaggaaa cgtctgtgtg agaacattgc cggccacctg aaggatgcac 1500

aaattttcat ccagaagaaa gcggtcaaga acttcactga ggtccaccct gactacggga 1560

gccacatcca ggctcttctg gacaagtaca atgctgagaa gcctaagaat gcgattcaca 1620

cctttgtgca gtccggatct cacttggcgg caagggagaa ggcaaatctg tgaggccggg 1680

gccctgcacc tgtgcagcga agcttagcgt tcatccgtgt aacccgctca tcactggatg 1740

aagattctcc tgtgctagat gtgcaaatgc aagctagtgg cttcaaaata gagaatccca 1800

ctttctatag cagattgtgt aacaatttta atgctatttc cccaggggaa aatgaaggtt 1860

aggatttaac agtcatttaa aaaaaaaatt tgttttgacg gatgattgga ttattcattt 1920

aaaatgatta gaaggcaagt ttctagctag aaatatgatt ttatttgaca aaatttgttg 1980

aaattatgta tgtttacata tcacctcatg gcctattata ttaaaatatg gctataaata 2040

tataaaaaga aaagataaag atgatctact cagaaatttt tatttttcta aggttctcat 2100

aggaaaagta catttaatac agcagtgtca tcagaagata acttgagcac cgtcatggct 2160

taatgtttat tcctgataat aattgatcaa attcattttt ttcactggag ttacattaat 2220

gttaattcag cactgatttc acaacagatc aatttgtaat tgcttacatt tttacaataa 2280

ataatctgta cgtaagaaca 2300

<210> 2
<211> 981
<212> DNA
<213> Homo sapiens

<400> 2

```
gtttggggcc agagtgggcg aggcgcggag gtctggccta taaagtagtc gcggagacgg      60
ggtgctggtt tgcgtcgtag tctcctgcag cgtctggggt ttccgttgca gtcctcggaa     120
ccaggacctc ggcgtggcct agcgagttat ggcgacgaag gccgtgtgcg tgctgaaggg     180
cgacggccca gtgcagggca tcatcaattt cgagcagaag gaaagtaatg gaccagtgaa     240
ggtgtgggga agcattaaag gactgactga aggcctgcat ggattccatg ttcatgagtt     300
tggagataat acagcaggct gtaccagtgc aggtcctcac tttaatcctc tatccagaaa     360
acacggtggg ccaaaggatg aagagaggca tgttggagac ttgggcaatg tgactgctga     420
caaagatggt gtggccgatg tgtctattga agattctgtg atctcactct caggagacca     480
ttgcatcatt ggccgcacac tggtggtcca tgaaaaagca gatgacttgg gcaaaggtgg     540
aaatgaagaa agtacaaaga caggaaacgc tggaagtcgt ttggcttgtg gtgtaattgg     600
gatcgcccaa taaacattcc cttggatgta gtctgaggcc ccttaactca tctgttatcc     660
tgctagctgt agaaatgtat cctgataaac attaaacact gtaatcttaa aagtgtaatt     720
gtgtgacttt ttcagagttg ctttaaagta cctgtagtga gaaactgatt tatgatcact     780
tggaagattt gtatagtttt ataaaactca gttaaaatgt ctgtttcaat gacctgtatt     840
ttgccagact taaatcacag atgggtatta aacttgtcag aatttctttg tcattcaagc     900
ctgtgaataa aaaccctgta tggcacttat tatgaggcta ttaaaagaat ccaaattcaa     960
actaaaaaaa aaaaaaaaaa a                                               981
```

<210> 3
<211> 921
<212> DNA
<213> Homo sapiens

<400> 3

cagttaaaag gaggcgcctg ctggcctccc cttacagtgc ttgttcgggg cgctccgctg 60
gcttcttgga caattgcgcc atgtgtgctg ctcggctagc ggcggcggcg gcggcggccc 120
agtcggtgta tgccttctcg gcgcgcccgc tggccggcgg ggagcctgtg agcctgggct 180
ccctgcgggg caaggtacta cttatcgaga atgtggcgtc cctctgaggc accacggtcc 240
gggactacac ccagatgaac gagctgcagc ggcgcctcgg accccggggc ctggtggtgc 300
tcggcttccc gtgcaaccag tttgggcatc aggagaacgc caagaacgaa gagattctga 360
attccctcaa gtacgtccgg cctggtggtg ggttcgagcc caacttcatg ctcttcgaga 420
agtgcgaggt gaacggtgcg ggggcgcacc ctctcttcgc cttcctgcgg gaggccctgc 480
cagctcccag cgacgacgcc accgcgctta tgaccgaccc caagctcatc acctggtctc 540
cggtgtgtcg caacgatgtt gcctggaact ttgagaagtt cctggtgggc cctgacggtg 600
tgcccctacg caggtacagc cgccgcttcc agaccattga catcgagcct gacatcgaag 660
ccctgctgtc tcaagggccc agctgtgcct agggcgcccc tcctaccccg gctgcttggc 720
agttgcagtg ctgctgtctc ggggggggttt tcatctatga gggtgtttcc tctaaaccta 780
cgagggagga acacctgatc ttacagaaaa taccacctcg agatgggtgc tggtcctgtt 840
gatcccagtc tctgccagac caaggcgagt ttccccacta ataaagtgcc gggtgtcagc 900
agaaaaaaaa aaaaaaaaaa a 921

<210> 4
<211> 942
<212> DNA
<213> Homo sapiens

<400> 4

gagcgctctg gagggcgtgg ccgtgggaaa ggaggcgcgg aaagccgacg cgcgtccatt 60

```
ggtcggctgg acgaggggag gagccgctgg ctcccagccc cgccgcgatg agcctcggcc      120
gcctttgccg cctactgaag ccggcgctgc tctgtggggc tctggccgcg cctggcctgg      180
ccgggaccat gtgcgcgtcc cgggacgact ggcgctgtgc gcgctccatg cacgagtttt      240
ccgccaagga catcgacggg cacatggtta acctggacaa gtaccggggc ttcgtgtgca      300
tcgtcaccaa cgtggcctcc cagtgaggca agaccgaagt aaactacact cagctcgtcg      360
acctgcacgc ccgatacgct gagtgtggtt tgcggatcct ggccttcccg tgtaaccagt      420
tcgggaagca ggagccaggg agtaacgaag agatcaaaga gttcgccgcg ggctacaacg      480
tcaaattcga tatgttcagc aagatctgcg tgaacgggga cgacgcccac ccgctgtgga      540
agtggatgaa gatccaaccc aagggcaagg gcatcctggg aaatgccatc aagtggaact      600
tcaccaagtt cctcatcgac aagaacggct gcgtggtgaa gcgctacgga cccatggagg      660
agcccctggt gatagagaag gacctgcccc actatttcta gctccacaag tgtgtggccc      720
cgcccgagcc cctgcccacg cccttggagc cttccaccgg cactcatgac ggcctgcctg      780
caaacctgct ggtggggcag acccgaaaat ccagcgtgca ccccgccgga ggaaggtccc      840
atggcctgct gggcttggct cggcgccccc acccctggct accttgtggg aataaacaga      900
caaattagcc tgctggaaaa aaaaaaaaaa aaaaaaaaaa aa                         942
```

<210> 5
<211> 1262
<212> DNA
<213> Homo sapiens

<400> 5

actctcgcga gatccctact ggctataaag gcagcgcccc ggagagctct tgcgcgtctt 60

gttcttgcct ggtgtcggtg gttagtttct gcgacttgtg ttgggactgg tgagtgtggg 120

cagtgcggcc cctgcggagt gaggcgcggc gcgcccttct tgcctgttgc ctcttcctcc 180

tcctgtccgg ggcccgcccg cgctcgggtg ggggtgctgt gatgcgtgag gcagccgggg 240

gaggcccgga gtccgagact gcttgagcgc tgcgcacacc cctctcgtgg gcccccacg 300

taggtgcggg aacctggttg aaccccaagc tgataggaag atgtcttcag gaaatgctaa 360

aattgggcac cctgccccca acttcaaagc cacagctgtt atgccagatg gtcagtttaa 420

agatatcagc ctgtctgact acaaaggaaa atatgttgtg ttcttctttt accctcttga 480

cttcaccttt gtgtgcccca cggagatcat tgctttcagt gatagggcag aagaatttaa 540

gaaactcaac tgccaagtga ttggtgcttc tgtggattct cacttctgtc atctagcatg 600

ggtcaataca cctaagaaac aaggaggact gggacccatg aacattcctt tggtatcaga 660

cccgaagcgc accattgctc aggattatgg ggtcttaaag gctgatgaag gcatctcgtt 720

caggggcctt tttatcattg atgataaggg tattcttcgg cagatcactg taaatgacct 780

ccctgttggc cgctctgtgg atgagacttt gagactagtt caggccttcc agttcactga 840

caaacatggg gaagtgtgcc cagctggctg gaaacctggc agtgatacca tcaagcctga 900

tgtccaaaag agcaaagaat atttctccaa gcagaagtga gcgctgggct gttttagtgc 960

caggctgcgg tgggcagcca tgagaacaaa acctcttctg tattttttt ttccattagt 1020

aaaacacaag acttcagatt cagccgaatt gtggtgtctt acaaggcagg cctttcctac 1080

agggggtgga gagaccagcc tttcttcctt tggtaggaat ggcctgagtt ggcgttgtgg 1140

gcaggctact ggtttgtatg atgtattagt agagcaaccc attaatcttt tgtagtttgt 1200

attaaacttg aactgagacc ttgatgagtc tttaaaaaaa aaaaaaaaaa aaaaaaaaaa 1260

aa 1262

<210> 6
<211> 1593
<212> DNA
<213> Homo sapiens

<400> 6

gcggtgccct tgcggcgcag ctggggtcgc ggccctgctc cccgcgcttt cttaaggccc 60

gcgggcggcg caggagcggc actcgtggct gtggtggctt cggcagcggc ttcagcagat 120

cggcggcatc agcggtagca ccagcactag cagcatgttg agccgggcag tgtgcggcac 180

cagcaggcag ctggctccgg ttttggggta tctgggctcc aggcagaagc acagcctccc 240

cgacctgccc tacgactacg gcgccctgga acctcacatc aacgcgcaga tcatgcagct 300

gcaccacagc aagcaccacg cggcctacgt gaacaacctg aacgtcaccg aggagaagta 360

ccaggaggcg ttggccaagg gagatgttac agcccagata gctcttcagc ctgcactgaa 420

gttcaatggt ggtggtcata tcaatcatag cattttctgg acaaacctca gccctaacgg 480

tggtggagaa cccaaagggg agttgctgga agccatcaaa cgtgactttg gttcctttga 540

caagtttaag gagaagctga cggctgcatc tgttggtgtc caaggctcag gttggggttg 600

gcttggtttc aataaggaac ggggacactt acaaattgct gcttgtccaa atcaggatcc 660

actgcaagga acaacaggcc ttattccact gctggggatt gatgtgtggg agcacgctta 720

ctaccttcag tataaaaatg tcaggcctga ttatctaaaa gctatttgga atgtaatcaa 780

ctgggagaat gtaactgaaa gatacatggc ttgcaaaaag taaaccacga tcgttatgct 840

gagtatgtta agctctttat gactgttttt gtagtggtat agagtactgc agaatacagt 900

aagctgctct attgtagcat ttcttgatgt tgcttagtca cttatttcat aaacaactta 960

atgttctgaa taatttctta ctaaacattt tgttattggg caagtgattg aaaatagtaa 1020

atgctttgtg tgattgaatc tgattggaca ttttcttcag agagctaaat tacaattgtc 1080

atttataaaa ccatcaaaaa tattccatcc atatactttg gggacttgta gggatgcctt 1140

tctagtccta ttctattgca gttatagaaa atctagtctt ttgccccagt tacttaaaaa 1200

taaaatatta acactttccc aagggaaaca ctcggctttc tatagaaaat tgcacttttt 1260

gtcgagtaat cctctgcagt gatacttctg gtagatgtca cccagtggtt tttgttaggt 1320

caaatgttcc tgtatagttt ttgcaaatag agctgtatac tgtttaaatg tagcaggtga 1380

actgaactgg ggtttgctca cctgcacagt aaaggcaaac ttcaacagca aaactgcaaa 1440

aaggtggttt ttgcagtagg agaaaggagg atgtttattt gcagggcgcc aagcaaggag 1500

aattgggcag ctcatgcttg agacccaatc tccatgatga cctacaagct agagtattta 1560

aaggcagtgg taaatttcag gaaagcagaa gtt 1593

<210> 7
<211> 1024
<212> DNA
<213> Homo sapiens

<400> 7

```
cttcctggct cctccttcct ccccacccct ctaataggct cataagtggg ctcaggcctc      60
tctgcggggc tcactctgcg cttcaccatg gctttcattg ccaagtcctt ctatgacctc     120
agtgccatca gcctggatgg ggagaaggta gatttcaata cgttccgggg cagggccgtg     180
ctgattgaga atgtggcttc gctctgaggc acaaccaccc gggacttcac ccagctcaac     240
gagctgcaat gccgctttcc caggcgcctg gtggtccttg gcttcccttg caaccaattt     300
ggacatcagg agaactgtca gaatgaggag atcctgaaca gtctcaagta tgtccgtcct     360
gggggtggat accagcccac cttcaccctt gtccaaaaat gtgaggtgaa tgggcagaac     420
gagcatcctg tcttcgccta cctgaaggac aagctcccct acccttatga tgacccattt     480
tccctcatga ccgatcccaa gctcatcatt tggagccctg tgcgccgctc agatgtggcc     540
tggaactttg agaagttcct catagggccg gagggagagc ccttccgacg ctacagccgc     600
accttcccaa ccatcaacat tgagcctgac atcaagcgcc tccttaaagt tgccatatag     660
atgtgaactg ctcaacacac agatctccta ctccatccag tcctgaggag ccttaggatg     720
cagcatgcct tcaggagaca ctgctggacc tcagcattcc cttgatatca gtccccttca     780
ctgcagagcc ttgcctttcc cctctgcctg tttccttttc ctctcccaac cctctggttg     840
gtgattcaac ttgggctcca agacttgggt aagctctggg ccttcacaga atgatggcac     900
cttcctaaac cctcatgggt ggtgtctgag aggcgtgaag ggcctggagc cactctgcta     960
gaagagacca ataaagggca ggtgtggaaa cggcaaaaaa aaaaaaaaaa aaaaaaaaaa    1020
aaaa                                                                 1024
```

<210> 8
<211> 1779
<212> DNA
<213> Homo sapiens

<400> 8

gtcgccccgg gacggggagg tggggagctg agggcaagtc gcgcccgccc ctgaaatccc 60
agccgcctag cgattggctg caagggtctc ggcttggccg cggattggtc acacccgagg 120
gcttgaaagg tggctgggag cgccggacac ctcagacgga cggtggccag ggatcaggca 180
gcggctcagg cgaccctgag tgtgccccca ccccgccatg gcccggctgc tgcaggcgtc 240
ctgcctgctt tccctgctcc tggccggctt cgtctcgcag agccggggac aagagaagtc 300
gaagatggac tgccatggtg gcataagtgg caccatttac gagtacggag ccctcaccat 360
tgatggggag gagtacatcc ccttcaagca gtatgctggc aaatacgtcc tctttgtcaa 420
cgtggccagc tactgaggcc tgacgggcca gtacattgaa ctgaatgcac tacaggaaga 480
gcttgcacca ttcggtctgg tcattctggg ctttccctgc aaccaatttg gaaaacagga 540
accaggagag aactcagaga tccttcctac cctcaagtat gtccgaccag gtggaggctt 600
tgtccctaat ttccagctct ttgagaaagg ggatgtcaat ggagagaaag agcagaaatt 660
ctacactttc ctaaagaact cctgtcctcc cacctcggag ctcctgggta catctgaccg 720
cctcttctgg gaacccatga aggttcacga catccgctgg aactttgaga agttcctggt 780
ggggccagat ggtataccca tcatgcgctg gcaccaccgg accacggtca gcaacgtcaa 840
gatggacatc ctgtcctaca tgaggcggca ggcagccctg gggtcaaga ggaagtaact 900
gaaggccgtc tcatcccatg tccaccatgt aggggaggga ctttgttcag gaagaaatcc 960
gtgtctccaa ccacactatc tacccatcac agaccccttt cctatcactc aaggccccag 1020
cctggcacaa atggatgcat acagttctgt gtactgccag gcatgtgggt gtgggtgcat 1080
gtgggtgttt acacacatgc ctacaggtat gcgtgattgt gtgtgtgtgc atgggtgtac 1140
agccacgtgt ctacctatgt gtctttctgg gaatgtgtac catctgtgtg cctgcagctg 1200
tgtagtgctg gacagtgaca accctttctc tccagttctc cactccaatg ataatagttc 1260
acttacacct aaacccaaag gaaaaaccag ctctaggtcc aattgttctg ctctaactga 1320
tacctcaacc ttggggccag catctcccac tgcctccaaa tattagtaac tatgactgac 1380
gtccccagaa gtttctgggt ctaccacact ccccaacccc ccactcctac ttcctgaagg 1440
gccctcccaa ggctacatcc ccaccccaca gttctccctg agagagatca acctccctga 1500
gatcaaccaa ggcagatgtg acagcaaggg ccacggaccc catggcaggg gtggcgtctt 1560
catgagggag gggcccaaag cccttgtggg cggacctccc ctgagcctgt ctgaggggcc 1620
agcccttagt gcattcaggc taaggcccct gggcagggat gccacccctg ctccttcgga 1680
ggacgtgccc tcacccctca ctggtccact ggcttgagac tcaccccgtc tgcccagtaa 1740
aagcctttct gcagcagctg aaaaaaaaaa aaaaaaaaa 1779

<210> 9

<211> 735
<212> DNA
<213> Homo sapiens

<400> 9

```
gcggtgtccg gcagtagagc tcgctgcaga tccgggctct gaccatgatt tggcgccgcg      60
cggcgctggc ggggacgcgg ctggtttgga gcaggagcgg ctcggcaggc tggcttgaca     120
gggcggcggg agctgcggga gctgcggcag ctgcggcctc tgggatggag agcaatacat     180
catcatcttt ggagaattta gcgacggcgc ctgtgaacca gatccaagaa acaatttctg     240
ataattgtgt ggtgattttc tcaaaaacat cctgttctta ctgtacaatg gcaaaaaagc     300
ttttccatga catgaatgtt aactataaag tggtggaact ggacctgctt gaatatggaa     360
accagttcca agatgctctt tacaaaatga ctggtgaaag aactgttcca agaatatttg     420
tcaatggtac ttttattgga ggtgcaactg acactcatag gcttcacaaa gaaggaaaat     480
tgctcccact agttcatcag tgttatttaa aaaaaagtaa gaggaaagaa tttcagtgat     540
gtttatacta ataagtttgc tagtacagtg tcagttattt aaagtggtaa tgcccgataa     600
tgtcttttaa atgtttgagg atgttttaaa tacatgcatt gtcttcacga agaagatgta     660
aaaataatga acaataaatt gcggtggaaa cctcaaaaaa aaaaaaaaaa aaaaaaaaaa     720
aaaaaaaaaa aaaaa                                                      735
```

<210> 10
<211> 827
<212> DNA
<213> Homo sapiens

<400> 10

gcagtggagg cggcccaggc ccgccttccg cagggtgtcg ccgctgtgcc gctagcggtg 60

ccccgcctgc tgcggtggca ccagccagga ggcggagtgg aagtggccgt ggggcgggta 120

tgggactagc tggcgtgtgc gccctgagac gctcagcggg ctatatactc gtcggtgggg 180

ccggcggtca gtctgcggca gcggcagcaa gacggtgcag tgaaggagag tgggcgtctg 240

gcggggtccg cagtttcagc agagccgctg cagccatggc cccaatcaag gtgggagatg 300

ccatcccagc agtggaggtg tttgaagggg agccagggaa caaggtgaac ctggcagagc 360

tgttcaaggg caagaagggt gtgctgtttg gagttcctgg ggccttcacc cctggatgtt 420

ccaaggttcg gctcctggct gatcccactg gggcctttgg gaaggagaca gacttattac 480

tagatgattc gctggtgtcc atctttggga atcgacgtct caagaggttc tccatggtgg 540

tacaggatgg catagtgaag gccctgaatg tggaaccaga tggcacaggc ctcacctgca 600

gcctggcacc caatatcatc tcacagctct gaggccctgg gccagattac ttcctccacc 660

cctccctatc tcacctgccc agccctgtgc tggggccctg caattggaat gttggccaga 720

tttctgcaat aaacacttgt ggtttgcggc caaaaaaaaa aaaaaaaaaa aaaaaaaaaa 780

aaaaaaaaaa aaaaaaaaaa aaaaaaaaaa aaaaaaaaaa aaaaaaa 827

<210> 11
<211> 3174
<212> DNA
<213> Homo sapiens

<400> 11

tcctcctggg tcttgcctag cggcgggcgc atgcttagtc accgtgaggc tgcgcttgcc 60

cggggcccgc gcccccctac cccggggacc gcccccgggc cgcccgcccc acttggcgcg 120

ccacttccgc gtgcatggcc ctgctgcccc gagccctgag cgccggcgcg ggaccgagct 180

ggcggcgggc ggcgcgcgcc ttccgaggct tcctgctgct tctgcccgag cccgcggccc 240

tcacgcgcgc cctctcccgt gccatggcct gcaggcagga gccgcagccg cagggcccgc 300

cgcccgctgc tggcgccgtg gcctcctatg actacctggt gatcgggggc ggctcgggcg 360

ggctggccag cgcgcgcagg gcggccgagc tgggtgccag ggccgccgtg gtggagagcc 420

acaagctggg tggcacttgc gtgaatgttg gatgtgtacc caaaaaggta atgtggaaca 480

cagctgtcca ctctgaattc atgcatgatc atgctgatta tggctttcca agttgtgagg 540

gtaaattcaa ttggcgtgtt attaaggaaa agcgggatgc ctatgtgagc cgcctgaatg 600

ccatctatca aaacaatctc accaagtccc atatagaaat catccgtggc catgcagcct 660

tcacgagtga tcccaagccc acaatagagg tcagtgggaa aaagtacacc gccccacaca 720

tcctgatcgc cacaggtggt atgccctcca cccctcatga gagccagatc cccggtgcca 780

gcttaggaat aaccagcgat ggatttttc agctggaaga attgcccggc cgcagcgtca 840

ttgttggtgc aggttacatt gctgtggaga tggcagggat cctgtcagcc ctgggttcta 900

agacatcact gatgatacgg catgataagg tacttagaag ttttgattca atgatcagca 960

ccaactgcac ggaggagctg gagaacgctg gcgtggaggt gctgaagttc tcccaggtca 1020

aggaggttaa aaagactttg tcgggcttgg aagtcagcat ggttactgca gttcccggta 1080

ggctaccagt catgaccatg attccagatg ttgactgcct gctctgggcc attgggcggg 1140

tcccgaatac caaggacctg agtttaaaca aactggggat tcaaaccgat gacaagggtc 1200

atatcatcgt agacgaattc cagaatacca acgtcaaagg catctatgca gttggggatg 1260

tatgtggaaa agctcttctt actccagttg caatagctgc tggccgaaaa cttgcccatc 1320

gactttttga atataaggaa gattccaaat tagattataa caacatccca actgtggtct 1380

tcagccaccc ccctattggg acagtgggac tcacggaaga tgaagccatt cataaatatg 1440

gaatagaaaa tgtgaagacc tattcaacga gctttacccc gatgtatcac gcagttacca 1500

aaaggaaaac aaaatgtgtg atgaaaatgg tctgtgctaa caaggaagaa aaggtggttg 1560

ggatccatat gcagggactt gggtgtgatg aaatgctgca gggttttgct gttgcagtga 1620

agatgggagc aacgaaggca gactttgaca acacagtcgc cattcaccct acctcttcag 1680

aagagctggt cacacttcgt tgagaaccag gagacacgtg tggcgggcag tgggacccat 1740

agatcttctg aaatgaaaca aataatcaca ttgacttact gtttgagttt tatgtatttc 1800

tttattttaa tcaggatctt ctgatagtgg aaatttttag tacataatag aacttattta 1860

tggagttaga aatttgtagt gttatccagg attgattttc atttgatcac atctcacagt 1920

aattaatatt ttcaagtttt tttttatta acagctctgt gctagttttt tttttctgtt 1980

ttagcctcat cccaaatata aagctttgtg aagtacaatt aacttaatgt acttgaatga 2040

atagaacttg ctactttttt tttttttttt tttgagacag agttttgctc tcattgccca 2100

ggctggagtg cggtggtgct atttcagctc accacaacct ctgcctcctg ggttcaagtg 2160

attctcctgc cttagcctcc cgaatagctg gaattacagg cacgcaccac catgcctgac 2220

taattttgta tttttagtag acatggggtt tctccatgtt ggtcaggctg gtctcaaact 2280

cccaccttca ggtgatccgc ccacctcggc ctcctgaggt gctgagatta caggcgtgag 2340

ccactgtgcc agcttgctaa ttttcacaga agttgatggc aattcttcac atgtaaacag 2400

tgccagtgca cagaaccttt atatattttt tgaagccagt actgtgctct gcatataaca 2460

aagctgcttc aaggatgaga cctttttcta aaagcatgta atgtgagaag ccggcctgcc 2520

ttattttctt ttttcttttt taatgattaa aaatagtttg tggcaaggca cggtggctca 2580

ggcctgtaat tctagcactt tgggaggccg aggcaggagg attacttgag cctacaagtt 2640

tgaggccagc atgcacagca tagcaagact gcatctctac agagagtaaa aaaaattacc 2700

cgagtgtggt gatgtgcatc tgtaatctca gctacttggg aggctgaggt gagaggatca 2760

cttgagcttg ggtgaggtga ggctgcagtg agtcctgatc atgctgctgc actcaatctt 2820

ggacaacaga gcaagaccct gtctcaaaaa aaaaaaaaaa aaatatatat atatatatat 2880

attattttta tgaggtgaag tgcatcaaac ttgggaaaga tttgaggagg ctgggaacct 2940

cctggaaaac cactccttga agaaagatat gagagacatt tagaagtgat tcctgctttc 3000

agaaggaggt ggattcaaat acatcaaaag tcccttcctc tgctaagtgt ttatagttca 3060

atgaataatt tcaatatttg tatgtgttct tgtcatttta tttttttctg aaaaacttcc 3120

aaaaatttga aaataaaatt acagcctttt cttcttataa aaaaaaaaaa aaaa 3174

<210> 12
<211> 1661
<212> DNA
<213> Homo sapiens

<400> 12

attgcattcc tgggcattgc taactagtga agtataccag atggaaatgt cttcgaagct 60

gtccctttaa aactcgagca agctaccagg caaactccgc ctccagggag gttccttatt 120

aaataggagc caactggctg ggtcggggct caatacccca agcaatacct gcaactgagg 180

attcttcccg gggagaccgc agcccatcgg catggctcaa gagtttgtga actgcaaaat 240

ccagcctggg aaggtggttg tgttcatcaa gcccacctgc ccgtactgca ggagggccca 300

agagatcctc agtcaattgc ccatcaaaca agggcttctg gaatttgtcg atatcacagc 360

caccaaccac actaacgaga ttcaagatta tttgcaacag ctcacgggag caagaacggt 420

gcctcgagtc tttattggta aagattgtat aggcggatgc agtgatctag tctctttgca 480

acagagtggg gaactgctga cgcggctaaa gcagattgga gctctgcagt aaccacagaa 540

caggccccat gctgacgtcc ctcctcaaga gctggatggc attgcaaatg atgacagcac 600

ttctggtgga tgaatttggg ggcacaaaca gctttttttcc tcttttggct cagtatttaa 660

aagtggacca acttgctctt aatcacaggg ccaagaaggt tgacgggcca tcttggtttt 720

cttctggatg tgctctttgg ttttcagaag actgtgacaa gttctggccc aggattcgct 780

cactgaccct caattgtcct ctttggcatg cgtttcttac tgttctccat gtgtcggcat 840

gtctctacct ctaagccagt gtttttcaac tatgtttatc cagactcctt ctccacaatg 900

atgaatccac agttggttat ctgctactgc ccattagcta aaatcatttt gctgcttgac 960

tttatggagt ttgtattatg aaatcagtgg gtattttgaa tgtgttcttt ctaactacat 1020

gcatctctcc actcaactcc accccatccc atcccacctt gaaaatcact gctctgaacc 1080

agtgttctcc accttgtcct ccacagatct cataggaaat gttcaacaat tctgtgaaag 1140

gtcacaggac ccaattggag aaatcatatg aaaagcatag ttggtcttgg tgtcatatgg 1200

atcagaggca caagtgcaga ggctgtggtc atgcggaaca ctctgttatt taagatggct 1260

atccagataa tcctgaacac tgtgtattta ttttatttag actaccagca aagattaaag 1320

catgaaatgt aaaacatctg ataaaactta cagcccccta caccaagagt gtatctgtga 1380

aagagctcct acactttgaa aacttaagaa tcccttatca tgaagtttgc ctgttctaga 1440

attgtaagat tgttaatttc cttcaatctc tagtgacaac acttaatttc ttttctaata 1500

aaaaaaacct atagatgatt cagtgatttt tgtccaattc atttgcatgt tctcaagaca 1560

ttaaggaatg ttatgcgaaa tacactaact taaaactgtg tttatatttg gccctgccat 1620

tataaataaa gacacgtgct gctgtcaaaa aaaaaaaaaa a 1661

<210> 13
<211> 1039
<212> DNA
<213> Homo sapiens

<400> 13

```
gctcgtccgc tccctccccc gcgccgtgca cgtcttggtt cgggccgggc ataaaaggct        60
tcgcggccca gggctcactt ggcgctgaga acgcgggtcc acgcgtgtga tcgtccgtgc       120
gtctagcctt tgcccacgca gctttcagtc atggcctccg gtaacgcgcg catcggaaag       180
ccagcccctg acttcaaggc cacagcggtg gttgatggcg ccttcaaaga ggtgaagctg       240
tcggactaca aagggaagta cgtggtcctc tttttctacc ctctggactt cacttttgtg       300
tgccccaccg agatcatcgc gttcagcaac cgtgcagagg acttccgcaa gctgggctgt       360
gaagtgctgg gcgtctcggt ggactctcag ttcacccacc tggcttggat caacaccccc       420
cggaaagagg gaggcttggg ccccctgaac atccccctgc ttgctgacgt gaccagacgc       480
ttgtctgagg attacggcgt gctgaaaaca gatgagggca ttgcctacag gggcctcttt       540
atcatcgatg gcaagggtgt ccttcgccag atcactgtta atgatttgcc tgtgggacgc       600
tccgtggatg aggctctgcg gctggtccag gccttccagt acacagacga gcatggggaa       660
gtttgtcccg ctggctggaa gcctggcagt gacacgatta agcccaacgt ggatgacagc       720
aaggaatatt tctccaaaca caattaggct ggctaacgga tagtgagctt gtgcccctgc       780
ctaggtgcct gtgctgggtg tccacctgtg cccccacctg ggtgccctat gctgacccag       840
gaaaggccag acctgcccct ccaaactcca cagtatggga ccctggaggg ctaggccaag       900
gccttctcat gcctccacct agaagctgaa tagtgacgcc ctcccccaag cccacccagc       960
cgcacacagg cctagaggta accaataaag tattagggaa aggtgtgaaa aaaaaaaaaa      1020
aaaaaaaaaa aaaaaaaaa                                                   1039
```

<210> 14
<211> 2799
<212> DNA
<213> Homo sapiens

<400> 14

```
acgtgtgcgg gagggaagca ggaagtgact gcgggagtgg agccggcgag agagtggcag        60
cgggggctga tggaagtgca gtgggggctg gagagggcac cctactgtat ccagcatgct       120
ccaaggccac agctctgtgt tccaggcctt gctggggacc ttcttcacct gggggatgac       180
agcagctggg gcagctctcg tgttcgtatt ctctagtgga cagaggcgga tcttagatgg       240
aagtcttggc tttgctgcag ggtcatgtt ggcagcttcc tattggtctc ttctggcccc        300
agcagttgag atggccacgt cctctggggg cttcggtgcc tttgccttct tccctgtggc       360
tgttggcttc acccttggag cggcttttgt ctacttggct gacctcctga tgcctcactt       420
gggtgcagca gaagaccccc agacgaccct ggcactgaac ttcggctcta cgttgatgaa       480
gaagaagtct gatcctgagg gtcccgcgct gctcttccct gagagtgaac tttccatccg       540
gataggtaga gctgggcttc tttcagacaa gagtgagaat ggtgaggcat atcagagaaa       600
gaaggcggca gccactggcc ttccagaggg tcctgctgtc cctgtgcctt ctcgagggaa       660
```

tctggcacag cccggcggca gcagctggag gaggatcgca ctgctcatct tggccatcac 720
tatacacaac gttccagagg gtctcgctgt tggagttgga tttggggcta tagaaaagac 780
ggcatctgct acctttgaga gtgccaggaa tttggccatt ggaatcggga tccagaattt 840
ccccgagggc ctggctgtca gccttccctt gcgaggggca ggcttctcca cctggagagc 900
tttctggtat gggcagctga gcggcatggt ggagcccctg gccggggtct ttggtgcctt 960
tgccgtggtg ctggctgagc ccatcctgcc ctacgctctg gcctttgctg ccggtgccat 1020
ggtctacgtg gtcatggacg acatcatccc cgaagcccag atcagtggta atgggaaact 1080
ggcatcctgg gcctccatcc tgggatttgt agtgatgatg tcactggacg ttggcctggg 1140
ctagggctga gacgcttcgg accccgggaa aggccatacg aagaaacagc agtggttggc 1200
ttctatggga caacaagctt ctttcttcac attaaaactt ttttccttcc tctcttcttc 1260
atctcattat cctgattgac tctgattata atagaaccat ttttactttg ctttgaggga 1320
gatttttgat ttaatgggga attttaaggt gtcatggaaa tacagattct ttgttttggc 1380
cactgaatgg actctctctt cagtgggatt atcaaggaac ttcagatcag ggaaatctcc 1440
acttcgggac cttctatctg cctcccaact cctcaaggtc acctatagaa gcgagctacc 1500
aaaagacgtc tcctaagcat tttggtggcc tagtgactca gggcagagtg gccagcacac 1560
ctctcatccg cccctcctgc tccatcactg ctgagcctct ccccatctag aatgttggaa 1620
ctggagcatc ataaagatag caagctacct tccaaggccg agccagccca gagaggagca 1680
tgtcttcctt tacctccccc taaggagata ctacatggga gggggacaca gaaaaaggga 1740
aggaaattgg ctagtctggc tttttttttt tttttttta aaggcaaaga ttgacattat 1800
tgaaggaaag gggatgagga caactgtgaa ctcacagtga gccctgtgga aagaagagac 1860
agacagagtg tgggtttgtt cggaggcctc tgctgtcaat ggattccagg agcaaggcca 1920
tttgtcgcgc tttccaaatt tcttaggcat ttattttgat aagtttatag ccatcatgtt 1980
tctaagagac ttggagacac cagcaaactg ctagaactca aactcttcaa ttactcaaag 2040
aaggagccat ttcagttaac tcaagtgaat gaaagagttt tggaatctgc tgtgggtcct 2100
tccctgttga ccatttggta acttataatc tgacaaaaac tcttgagctg caacaggcct 2160
tgccagaggg ctcaggatgg gaaaggaaga aggggatagg aaaagaagag gtaattttac 2220
atttcccctt taaagtaaat tttagccaac tcatcattct gaaatgtccc tataaagaat 2280
gagtcgaact agaccagaag ccagcctact ccttcttaca tagcttctcc aacaggggta 2340
gcaatgacct gtccacttca aacacagata aggcctgcca tcctcattgg ttaaaggcac 2400
acgtgagact ttcagtgggc tctgctgaga aggaaggcag cccaggagtc aggtatgcag 2460
gcattgcatt gtcagtgtct gctctcagag tttacacatt caattgcttc caagggtgaa 2520

tctcctgctc tgtgaatgct atcagacccc aaaggccaac cttgggctgg gtctatgtac 2580

gttcttccga agcactgatg atcaaaattg aagacacatt cagaggtttg attggttgag 2640

attaactggt gtggtggttg gtgtatgtat gttttatttt tatgtctttg tatgtagttc 2700

tacataatgc aaattgtgct ttctgatgga caagacctca taactgtgat taatatcaat 2760

aaaaagggga tgttgtggat gaaaaaaaaa aaaaaaaaa 2799

<210> 15
<211> 508
<212> DNA
<213> Homo sapiens

<400> 15

tttggtgctt tggatccatt tccatcggtc cttacagccg ctcgtcagac tccagcagcc 60

aagatggtga agcagatcga gagcaagact gcttttcagg aagccttgga cgctgcaggt 120

gataaacttg tagtagttga cttctcagcc acgtggtgtg ggccttgcaa aatgatcaag 180

cctttctttc attccctctc tgaaaagtat tccaacgtga tattccttga agtagatgtg 240

gatgactgtc aggatgttgc ttcagagtgt gaagtcaaat gcatgccaac attccagttt 300

tttaagaagg gacaaaaggt gggtgaattt tctggagcca ataaggaaaa gcttgaagcc 360

accattaatg aattagtcta atcatgtttt ctgaaaatat aaccagccat tggctattta 420

aaacttgtaa ttttttttaat ttacaaaaat ataaaatatg aagacataaa cccagttgcc 480

atctgcgtga caataaaaca ttaatgct 508

<210> 16
<211> 1591
<212> DNA
<213> Homo sapiens

<400> 16

```
ccctgcgtct ctgcccgccc cgtggcgccc gagtgcactg aagatggcgg ctgctgtagg      60
acggttgctc cgagcgtcgg ttgcccgaca tgtgagtgcc attccttggg gcatttctgc     120
cactgcagcc ctcaggcctg ctgcatgtgg aagaacgagc ttgacaaatt tattgtgttc     180
tggttccagt caagcaaaat tattcagcac cagttcctca tgccatgcac ctgctgtcac     240
ccagcatgca ccctatttta agggtacagc cgttgtcaat ggagagttca aagacctaag     300
ccttgatgac tttaagggga aatatttggt gcttttcttc tatcctttgg atttcacctt     360
tgtgtgtcct acagaaattg ttgcttttag tgacaaagct aacgaatttc acgacgtgaa     420
ctgtgaagtt gtcgcagtct cagtggattc ccactttagc catcttgcct ggataaatac     480
accaaggaag aatggtggtt tgggccacat gaacatcgca ctcttgtcag acttaactaa     540
gcagatttcc cgagactacg gtgtgctgtt agaaggttct ggtcttgcac taagaggtct     600
cttcataatt gaccccaatg gagtcatcaa gcatttgagc gtcaacgatc tcccagtggg     660

ccgaagcgtg gaagaaaccc tccgcttggt gaaggcgttc cagtatgtag aaacacatgg     720
agaagtctgc ccagcgaact ggacaccgga ttctcctacg atcaagccaa gtccagctgc     780
ttccaaagag tactttcaga aggtaaatca gtagatcacc catgtgtatc tgcaccttct     840
caactgagag aagaaccaca gttgaaacct gcttttatca ttttcaagat ggttatttgt     900
agaaggcaag gaaccaatta tgcttgtatt cataagtatt actctaaatg ttttgttttt     960
gtaattctgg ctaagacctt ttaaacatgg ttagttgcta gtacaaggaa tcctttattg    1020
gtaacatctt ggtggctggc tagctagttt ctacagaaca taatttgcct ctatagaagg    1080
ctattcttag atcatgtctc aatggaaaca ctcttctttc ttagccttac ttgaatcttg    1140
cctataataa agtagagcaa cacacattga aagcttctga tcaacggtcc tgaaattttc    1200
atcttgaatg tctttgtatt aaactgaatt ttcttttaag ctaacaaaga tcataatttt    1260
caatgattag ccgtgtaact cctgcaatga atgtttatgt gattgaagca aatgtgaatc    1320
gtattatttt aaaaagtggc agagtgactt aactgatcat gcatgatccc tcatccctga    1380
aattgagttt atgtagtcat tttacttatt ttattcatta gctaactttg tctatgtata    1440
tttctagata ttgattagtg taatcgatta taaaggatat ttatcaaatc cagggattgc    1500
attttgaaat tataattatt ttctttgctg aagtattcat tgtaaaacat acaaaataaa    1560
catattttaa aacatttgca ttttaccacc a                                   1591
```

<210> 17
<211> 1246
<212> DNA
<213> Homo sapiens

<400> 17

gtctttgccc tcgcgacgcc gccacctccg gaacaagcca tggtggcggc gacggtggca 60

gcggcgtggc tgctcctgtg ggctgcggcc tgcgcgcagc aggagcagga cttctacgac 120

ttcaaggcgg tcaacatccg gggcaaactg gtgtcgctgg agaagtaccg cggatcggtg 180

tccctggtgg tgaatgtggc cagcgagtgc ggcttcacag accagcacta ccgagccctg 240

cagcagctgc agcgagacct gggcccccac cactttaacg tgctcgcctt cccctgcaac 300

cagtttggcc aacaggagcc tgacagcaac aaggagattg agagctttgc ccgccgcacc 360

tacagtgtct cattccccat gtttagcaag attgcagtca ccggtactgg tgcccatcct 420

gccttcaagt acctggccca gacttctggg aaggagccca cctggaactt ctggaagtac 480

ctagtagccc cagatggaaa ggtggtaggg gcttgggacc caactgtgtc agtggaggag 540

gtcagacccc agatcacagc gctcgtgagg aagctcatcc tactgaagcg agaagactta 600

taaccaccgc gtctcctcct ccaccacctc atcccgccca cctgtgtggg gctgaccaat 660

gcaaactcaa atggtgcttc aaagggagag acccactgac tctccttcct ttactcttat 720

gccattggtc ccatcattct tgtgggggaa aaattctagt attttgatta tttgaatctt 780

acagcaacaa ataggaactc ctggccaatg agagctcttg accagtgaat caccagccga 840

tacgaacgtc ttgccaacaa aaatgtgtgg caaatagaag tatatcaagc aataatctcc 900

cacccaaggc ttctgtaaac tgggaccaat gattacctca tagggctgtt gtgaggatta 960

ggatgaaata cctgtgaaag tgcctaggca gtgccagcca aataggaggc attcaatgaa 1020

catttttgc atataaacca aaaaataact tgttatcaat aaaaacttgc atccaacatg 1080

aatttccagc cgatgataat ccaggccaaa ggtttagttg ttgttatttc ctctgtatta 1140

ttttcttcat tacaaaagaa atgcaagttc attgtaacaa tccaaacaat acctcacgat 1200

ataaaataaa aatgaaagta tcctcctcaa aaaaaaaaaa aaaaaa 1246

<210> 18
<211> 1342
<212> DNA
<213> Homo sapiens

<400> 18

```
gaggaagtga cgacaggcgt gcccttgaca ggcagggagg gctaggctgt gcatccctcc      60
gctcgcattg cagggagatg gctcagcgac ttcttctgag gaggttcctg gcctctgtca     120
tctccaggaa gccctctcag ggtcagtggc cacccctcac ttccagagcc ctgcagaccc     180
cacaatgcag tcctggtggc ctgactgtaa cacccaaccc agcccggaca atatacacca     240
cgaggatctc cttgacaacc tttaatatcc aggatggacc tgactttcaa gaccgagtgg     300
tcaacagtga gacaccagtg gttgtggatt tccacgcaca gtggtgtgga ccctgcaaga     360
tcctggggcc gaggttagag aagatggtgg ccaagcagca cgggaaggtg gtgatggcca     420
aggtggatat tgatgaccac acagacctcg ccattgagta tgaggtgtca gcggtgccca     480
ctgtgctggc catgaagaat ggggacgtgg tggacaagtt tgtgggcatc aaggatgagg     540
atcagttgga ggccttcctg aagaagctga ttggctgaca agcagggatg agtcctggtt     600
cccttgcccg cgtgggaccc caatagaact cagcccttcc atgccagccc ttcctgctgc     660
ctccctcctg tctggctcct ggggcccatg cttagagccc aggctccagc cctgagtgct     720
tccgagctgg cggactgccc aggggccatc agaggatggt ggtgctgctg ctgatccggg     780
gaccgctgtc ttccctccca tacgcctttc atccctcctt ctagggccta tggcagttct     840
cccaggatgt gtggcgagag cctgggccag cccacagcgt tcctagtcag gcagccacac     900
cttggtcctc atcttggtcc cttccaatct gaaacctcgt gcctggctcg tctgccacct     960
acatttctct ttccagctgc tgttttgtaa aaagaaaaag aaaaaagaag cccaaactag    1020
tgagagtaat atctaattat ctcatttttt gtaggtctgt gataaagaac ttagtcatcc    1080
cttccacctc ctactgtgaa gaacagaccc tgggtcccac actgaaatcc cctctagtca    1140
cccattccca ccccccaggg agctgcctcc caggcagggg gtgcagaaaa tgattgatgg    1200
gctggggaac cctggagagc ctcgactccg gaagtctcaa ggtgcctcct cctctcctta    1260
gctggcccgt tggttttctg agcaggggc tgaactgtga acaagtcaga caaataaagc     1320
aagggtctgc accatcaaaa aa                                             1342
```

<210> 19
<211> 921
<212> **DNA**
<213> Homo sapiens

<400> 19

gcggcgctcg cgccaaggga cgtgtttctg cgctcgcgtg gtcatggagg cgctgccgct 60
gctagccgcg acaactccgg accacggccg ccaccgaagg ctgcttctgc tgccgctact 120
gctgttcctg ctgccggctg gagctgtgca gggctgggag acagaggaga ggccccggac 180
tcgcgaagag gagtgccact tctacgcggg tggacaagtg tacccgggag aggcatcccg 240
ggtatcggtc gccgaccact ccctgcacct aagcaaagcg aagatttcca agccagcgcc 300
ctactgggaa ggaacagctg tgatcgatgg agaatttaag gagctgaagt taactgatta 360
tcgtgggaaa tacttggttt tcttcttcta cccacttgat ttcacatttg tgtgtccaac 420
tgaaattatc gcttttggcg acagacttga agaattcaga tctataaata ctgaagtggt 480
agcatgctct gttgattcac agtttaccca tttggcctgg attaataccc ctcgaagaca 540
aggaggactt gggccaataa ggattccact tctttcagat ttgacccatc agatctcaaa 600
ggactatggt gtatacctag aggactcagg ccacactctt agaggtctct tcattattga 660
tgacaaagga atcctaagac aaattactct gaatgatctt cctgtgggta gatcagtgga 720
tgagacacta cgtttggttc aagcattcca gtacactgac aaacacggag aagtctgccc 780
tgctggctgg aaacctggta gtgaaacaat aatcccagat ccagctggaa agctgaagta 840
tttcgataaa ctgaattgag aaatacttct tcaagttatg atgcttgaaa gttctcaata 900
aagttcacgg tttcattacc a 921

<210> 20
<211> 1200
<212> DNA
<213> Homo sapiens

<400> 20

cagttaaaag gaggcgcctg ctggcctccc cttacagtgc ttgttcgggg cgctccgctg 60
gcttcttgga caattgcgcc atgtgtgctg ctcggctagc ggcggcggcg gcggcggccc 120
agtcggtgta tgccttctcg gcgcgcccgc tggccggcgg ggagcctgtg agcctgggct 180
ccctgcgggg caaggtacta cttatcgaga atgtggcgtc cctctgaggc accacggtcc 240

gggactacac ccagatgaac gagctgcagc ggcgcctcgg accccggggc ctggtggtgc 300

tcggcttccc gtgcaaccag tttgggcatc aggtgcgccg ggcggagcgg ggcggggcgg 360

gggcggacgt gcagtagtgg ctgggggcgc cggcggtgtg ctggtgggtg ccgtcggctc 420

catgcgcgga gagtctggct actctctcgt ttcctttctg ttgctcgtag ctgctgaaat 480

tcctctccgc ccttgggatt gcgcatggag ggcaaaatcc cggtgactca tagaaaatct 540

cccttgtttg tggttagaac gtttctctcc tcctcttgac cccgggttct agctgccctt 600

ctctcctgta ggagaacgcc aagaacgaag agattctgaa ttccctcaag tacgtccggc 660

ctggtggtgg gttcgagccc aacttcatgc tcttcgagaa gtgcgaggtg aacggtgcgg 720

gggcgcaccc tctcttcgcc ttcctgcggg aggccctgcc agctcccagc gacgacgcca 780

ccgcgcttat gaccgacccc aagctcatca cctggtctcc ggtgtgtcgc aacgatgttg 840

cctggaactt tgagaagttc ctggtgggcc ctgacggtgt gcccctacgc aggtacagcc 900

gccgcttcca gaccattgac atcgagcctg acatcgaagc cctgctgtct caagggccca 960

gctgtgccta gggcgcccct cctaccccgg ctgcttggca gttgcagtgc tgctgtctcg 1020

ggggggtttt catctatgag ggtgtttcct ctaaacctac gagggaggaa cacctgatct 1080

tacagaaaat accacctcga gatgggtgct ggtcctgttg atcccagtct ctgccagacc 1140

aaggcgagtt tccccactaa taaagtgccg ggtgtcagca gaaaaaaaaa aaaaaaaaaa 1200

<210> 21
<211> 1274
<212> DNA
<213> Homo sapiens

<400> 21

gcttctgtct ggcggcggca gcatggcggc gggggcggct gaggcagctg tagcggccgt 60
ggaggaggtc ggctcagccg ggcagtttga ggagctgctg cgcctcaaag ccaagtccct 120
ccttgtggtc catttctggg caccatgggc tccacagtgt gcacagatga acgaagttat 180
ggcagagtta gctaaagaac tccctcaagt ttcatttgtg aagttggaag ctgaaggtgt 240
tcctgaagta tctgaaaaat atgaaattag ctctgttccc acttttctgt tttcaagaa 300
ttctcagaaa atcgaccgat tagatggtgc acatgcccca gagttgacca aaaaagttca 360
gcgacatgca tctagtggct ccttcctacc cagcgctaat gaacatctta aagaagatct 420
caaccttcgc ttgaagaaat tgactcatgc tgccccctgc atgctgttta tgaaaggaac 480
tcctcaagaa ccacgctgtg gtttcagcaa gcagatggtg gaaattcttc acaaacataa 540
tattcagttt agcagttttg atatcttctc agatgaagag gttcgacagg gactcaaagc 600
ctattccagt tggcctacct atcctcagct ctatgtttct ggagagctca taggaggact 660
tgatataatt aaggagctag aagcatctga agaactagat acaatttgtc ccaaagctcc 720
caaattagag gaaaggctca aagtgctgac aaataaagct tctgtgatgc tctttatgaa 780
aggaaacaaa caggaagcaa aatgtggatt cagcaaacaa attctggaaa tactaaatag 840
tactggtgtt gaatatgaaa cattcgatat attggaggat gaagaagttc ggcaaggatt 900
aaaagcttac tcaaattggc caacataccc tcagctgtat gtgaaagggg agctggtggg 960
aggattggat attgtgaagg aactgaaaga aaatggtgaa ttgctgccta tactgagagg 1020
agaaaattaa taaatcttaa acttggtgcc caactattgt aagaaatatt taattacatt 1080
gggagcagtt catgatttag tcctcagaaa tggactagga atagaaaatt cctgctttct 1140
cagttacatg ttttgtgtat ttcacaatgt cgtgctaaat aaatgtatgt tacatttttt 1200
tcccaccaaa aatagaatgc aataaacatc ttcaaattat taacgaaaaa aaaaaaaaaa 1260
aaaaaaaaaa aaaa 1274

<210> 22
<211> 710
<212> DNA
<213> Homo sapiens

<400> 22

gctcgtccgc tccctccccc gcgccgtgca cgtcttggtt cgggccgggc ataaaaggct 60
tcgcggccca gggctcactt ggcgctgaga acgcgggtcc acgcgtgtga tcgtccgtgc 120
gtctagcctt tgcccacgca gctttcagtc atggcctccg gtaacgcgcg catcggaaag 180
ccagcccctg acttcaaggc cacagcggtg gttgatggcg ccttcaaaga ggtgaagctg 240
tcggactaca aagggaagta cgtggtcctc tttttctacc ctctggactt cacttttgtg 300
tgccccaccg agatcatcgc gttcagcaac cgtgcagagg acttccgcaa gctgggctgt 360
gaagtgctgg gcgtctcggt ggactctcag ttcacccacc tggcttggta tgagcagggg 420
ccaaagaggg aggttgcagc taagctcaca ccctcaggtc ctagcagtgt ggcttcgtgg 480
ccattgctca acctctggaa cctgcgtttc cccatcgtga aaataatgga aacattgccg 540
cccaagtctt taaggatgat gacagtaatt agcatttgac aactagttgc ctggtatata 600
gagttgcaga tgcaactcag atgcaactct atctactcta tgtacttagt tcccaggagg 660
gaggctgtgc tgccctattt catgaagatg gaaactccag ttcaccgaag 710

<210> 23
<211> 1715
<212> DNA
<213> Homo sapiens

<400> 23

gaaccaaccg gttgcttgct gtcccagcgg cgccccctca tcaccgtcgc catgcccgga 60
ggtctgcttc tcggggacgt ggctcccaac tttgaggcca ataccaccgt cggccgcatc 120
cgtttccacg actttctggg agactcatgg ggcattctct tctcccaccc tcgggacttt 180

accccagtgt gcaccacaga gcttggcaga gctgcaaagc tggcaccaga atttgccaag 240

aggaatgtta agttgattgc cctttcaata gacagtgttg aggaccatct tgcctggagc 300

aaggatatca atgcttacaa ttgtgaagag cccacagaaa agttaccttt tcccatcatc 360

gatgatagga atcgggagct tgccatcctg ttgggcatgc tggatccagc agagaaggat 420

gaaaagggca tgcctgtgac agctcgtgtg gtgtttgttt ttggtcctga taagaagctg 480

aagctgtcta tcctctaccc agctaccact ggcaggaact ttgatgagat tctcagggta 540

gtcatctctc tccagctgac agcagaaaaa agggttgcca ccccagttga ttggaaggat 600

ggggatagtg tgatggtcct tccaaccatc cctgaagaag aagccaaaaa acttttcccg 660

aaaggagtct tcaccaaaga gctcccatct ggcaagaaat acctccgcta cacaccccag 720

ccttaagtct cttggagaag ctggtgctgt gagccagagg atgtcagctg ccaattgtgt 780

tttcctgcag caattccata aacacatcct ggtgtcatca cagccaaggt ttttaggttg 840

ctataccaat ggcttattaa atgaaaatgg cactaaaagt ttcttgagat tctttatact 900

ctctgccttc agcaatcaat tccattcata catcagcact ctgctggttc tgtttgaaat 960

atgttctgta tttaaaactc aaatcttgtt ggatctctgc agggcttgtg accaatgaag 1020

tcatatttgt tgatggttga caaagcttgc ttcactccat cagagaatga ctatcaattt 1080

tttttaact gtcctatcac gtcctctcct gtcacccatt ttgaagagtg gcagaacttg 1140

aagttcaact tcctctgtaa atatccaagt ataaagccca ggaacttcta gaataaccca 1200

gatgcgcttt aattttttt aatatgtttt gatcacagaa cttctagaat aacccagatg 1260

ctctttcata ttcttttaat acatcttgat cacagctggg ggaaaaaaag ctttttaatt 1320

ctataccttc ctagtagata agtgaagagc agggaaagag acctttaaat attttgctat 1380

aaaaaaattt gtgataagtt tctatcaaaa tggggagatt gcagaaaagg cttcccttgg 1440

ctcccaagga ggtgtagcag gtgtgagcaa tattagtgcc atgtgccttt cacacagggt 1500

ttgcatttat cagtctgttt tccgatgatg tgtacatgaa agagtacacc atgtgaagag 1560

aagagagaat gattgaaaat gttttagtat agaactcttc ttgcagtggg ttgctatttt 1620

ctagatttta ctttttaggg aacaaaataa aatcctttgt taaaactggg aaaaaaaaaa 1680

aaaaaaaaaa aaaaaaaaaa aaaaaaaaaa aaaaa 1715

<210> 24
<211> 1182
<212> DNA
<213> Homo sapiens

<400> 24

aatgagggcc tccagggggc gggtcggact gccgcgggcc ggggagcgct ctgggtggcc 60

agctgtgggc ccgggccgtc gtgggctccg gcttgcgtgc ggagatgagc gggtccctcg 120

gccgagctgc ggcggctctg ctccgctggg ggcgcggcgc gggcggcggt ggcctttggg 180

gtccgggcgt gcgggcggcg ggctcgggcg cgggcggcgg cggctcggcg gagcagttgg 240

acgcgctggt gaagaaggac aaggtggtgg tcttcctcaa ggggacgccg gagcagcccc 300

agtgcggctt cagcaacgcc gtggtgcaga tcctgcggct gcacggcgtc cgcgattacg 360

cggcctacaa cgtgctggac gacccggagc tccgacaagg cattaaagac tattccaact 420

ggcccaccat cccgcaagtg tacctcaatg gcgagtttgt agggggctgt gacattcttc 480

tgcagatgca ccagaatggg gacttggtgg aagaactgaa aaagctgggg atccactccg 540

ccctttaga tgaaaagaaa gaccaagact ccaagtgagg gcggccaagt cctcgctgag 600

cagagaggga gccgttcatg tcagagactc actgccagaa aagccttacc cattttggtt 660

ttcactattg agaccgcaac tgcttgcact gatcattttg gttcgtgagc agttggtgat 720

tttagttggt ctggtgttcg ggctaagaat attttattgt ggacttaatt acaaccactg 780

cactgtaatg attcaatgct gtattatgat attgctgtaa acaaaattca ttcttatatt 840

gtcacttatt ctttgcctga ttcagaagtt aaataggagc tttggaatca ttattcatga 900

cccctctgca aatgtgtcag tctccaaaga gagtatctcc ccccaaattt tgtgtagctt 960

cttttgttat ggaaaatggt gaacaaaaaa agaaactgtg ataactgggg cgttgttttt 1020

taaaataaac tccagcacag ggatgctgtg catgcctgag ttgattccga agtgcatatg 1080

tctgtaagga tttggagtgc ctgcagtgtt ttatgtgtgg gaagtaaggg tgagtctcat 1140

attcttctat taaatttgcc acaagaattg caaaaaaaaa aa 1182

<210> 25
<211> 1170
<212> DNA
<213> Homo sapiens

<400> 25

aaggctatta ttaccaccac tgagtggctt aaataatcct gtcaacagca atcgcccatt 60
tccaaagcca tggtgaaaca tctctgtgct aatttctttt gttttgtttc ctaatttttt 120
ttttttggca ggtggtggga aataatcttt gtcttctttg gagtaaacct tcaacaccgg 180
atttttttctt ttaattatgg atgtaaaccc caatatcccc ataatttaca ttgggtctcg 240
accaattgcc taattataag aggatatatt taggctctta tttcatccac acaaaaactt 300
gtgtaacagg tagttggaaa catctgaggc accactttga ttctgttttg gatggtcatg 360
tttttttctcc tccgtttccc cagcatgtct gccaccatcc tcatgcactg cttccaagtg 420
cctgggagcc tttatgagcg tccctaaacc taaaagaatc cagaggcggg gctcggatga 480
accctcgaga taagcaagtg agccgcttct cccctctaaa ggatgtttac acgtgggtgg 540
cactcgctgg aatccagcgc tcgggcagcc ctgggaggac gcgctcagct gcgaggagga 600
tggagagcaa tacatcatca tctttggaga atttagcgac ggcgcctgtg aaccagatcc 660
aagaaacaat ttctgataat tgtgtggtga ttttctcaaa aacatcctgt tcttactgta 720
caatggcaaa aaagcttttc catgacatga atgttaacta taaagtggtg gaactggacc 780
tgcttgaata tggaaaccag ttccaagatg ctctttacaa aatgactggt gaaagaactg 840
ttccaagaat atttgtcaat ggtactttta ttggaggtgc aactgacact cataggcttc 900
acaaagaagg aaaattgctc ccactagttc atcagtgtta tttaaaaaaa agtaagagga 960
aagaatttca gtgatgttta tactaataag tttgctagta cagtgtcagt tatttaaagt 1020
ggtaatgccc gataatgtct tttaaatgtt tgaggatgtt ttaaatacat gcattgtctt 1080
cacgaagaag atgtaaaaat aatgaacaat aaattgcggt ggaaacctaa aaaaaaaaaa 1140
aaaaaaaaaa aaaaaaaaaa aaaaaaaaaa 1170

<210> 26
<211> 964
<212> DNA
<213> Homo sapiens

<400> 26

```
gagcgctctg gagggcgtgg ccgtgggaaa ggaggcgcgg aaagccgacg cgcgtccatt        60
ggtcggctgg acgaggggag gagccgctgg ctcccagccc cgccgcgatg agcctcggcc       120
gcctttgccg cctactgaag ccggcgctgc tctgtggggc tctggccgcg cctggcctgg       180
ccgggaccat gtgcgcgtcc cgggacgact ggcgctgtgc gcgctccatg cacgagtttt       240
ccgccaagga catcgacggg cacatggtta acctggacaa gtaccggggc ttcgtgtgca       300
tcgtcaccaa cgtggcctcc cagtgaggca agaccgaagt aaactacact cagctcgtcg       360
acctgcacgc ccgatacgct gagtgtggtt tgcggatcct ggccttcccg tgtaaccagt       420
tcgggaagca ggagccaggg agtaacgaag agatcaaaga gttcgccgcg ggctacaacg       480
tcaaattcga tatgttcagc aagatctgcg tgaacgggga cgacgcccac ccgctgtgga       540
agtggatgaa gatccaaccc aagggcaagg gcatcctggg aaatgccatc aagtggaact       600
tcaccaagtt tggacaccgt ctctccacag ttcctcatcg acaagaacgg ctgcgtggtg       660
aagcgctacg gacccatgga ggagcccctg gtgatagaga aggacctgcc ccactatttc       720
tagctccaca agtgtgtggc cccgcccgag cccctgccca cgcccttgga gccttccacc       780
ggcactcatg acggcctgcc tgcaaacctg ctggtggggc agacccgaaa atccagcgtg       840
caccccgccg gaggaaggtc ccatggcctg ctgggcttgg ctcggcgccc ccacccctgg       900
ctaccttgtg ggaataaaca gacaaattag cctgctggaa aaaaaaaaaa aaaaaaaaaa       960
aaaa                                                                    964
```

<210> 27
<211> 1031
<212> DNA
<213> Homo sapiens

<400> 27

agtcctgact acggcctccg ggccctttgt ccccgctagc ggcgctcggg gtgggggagc 60

caggaggggc gggagacggg cgggtatggg ccgcgcgggc gcaggctccc ccgggcgccg 120

caggcagcgg tgccagagcc ggggcaggcg gcggccgcga gcccctcggc ggcggaaggc 180

cccagcgtgc aggcgcagga gggcgcggcg ccggcggaag aagccctgtc cccgcagctt 240

gcgaccggag atccacgaat gtcccaagtc ccaggacccg tgcgcgtccc gggacgactg 300

gcgctgtgcg cgctccatgc acgagttttc cgccaaggac atcgacgggc acatggttaa 360

cctggacaag taccggggct tcgtgtgcat cgtcaccaac gtggcctccc agtgaggcaa 420

gaccgaagta aactacactc agctcgtcga cctgcacgcc cgatacgctg agtgtggttt 480

gcggatcctg gccttcccgt gtaaccagtt cgggaagcag gagccaggga gtaacgaaga 540

gatcaaagag ttcgccgcgg gctacaacgt caaattcgat atgttcagca agatctgcgt 600

gaacggggac gacgcccacc cgctgtggaa gtggatgaag atccaaccca agggcaaggg 660

catcctggga aatgccatca agtggaactt caccaagttc ctcatcgaca agaacggctg 720

cgtggtgaag cgctacggac ccatggagga gcccctggtg atagagaagg acctgcccca 780

ctatttctag ctccacaagt gtgtggcccc gcccgagccc ctgcccacgc ccttggagcc 840

ttccaccggc actcatgacg gcctgcctgc aaacctgctg gtggggcaga cccgaaaatc 900

cagcgtgcac ccgccggag gaaggtccca tggcctgctg ggcttggctc ggcgccccca 960

cccctggcta ccttgtggga ataaacagac aaattagcct gctggaaaaa aaaaaaaaaa 1020

aaaaaaaaaa a 1031

<210> 28
<211> 1236
<212> DNA
<213> Homo sapiens

<400> 28

actctcgcga gatccctact ggctataaag gcagcgcccc ggagagctct tgcgcgtctt 60

gttcttgcct ggtgtcggtg gttagtttct gcgacttgtg ttgggactgg tgagtgtggg 120

cagtgcggcc cctgcggagt gaggcgcggc gcgcccttct tgcctgttgc ctcttcctcc 180

tcctgtccgg ggcccgcccg cgctcgggtg ggggtgctgt gatgcgtgag gcagccgggg 240

gaggcccgga gtccgagact gcttgagcgc tgcgcacacc cctctcgtgg gccccccacg 300

tagctgatag gaagatgtct tcaggaaatg ctaaaattgg gcaccctgcc cccaacttca 360

aagccacagc tgttatgcca gatggtcagt ttaaagatat cagcctgtct gactacaaag 420

gaaaatatgt tgtgttcttc ttttaccctc ttgacttcac ctttgtgtgc cccacggaga 480
tcattgcttt cagtgatagg gcagaagaat ttaagaaact caactgccaa gtgattggtg 540
cttctgtgga ttctcacttc tgtcatctag catgggtcaa tacacctaag aaacaaggag 600
gactgggacc catgaacatt cctttggtat cagacccgaa gcgcaccatt gctcaggatt 660
atggggtctt aaaggctgat gaaggcatct cgttcagggg cctttttatc attgatgata 720
agggtattct tcggcagatc actgtaaatg acctccctgt tggccgctct gtggatgaga 780
ctttgagact agttcaggcc ttccagttca ctgacaaaca tggggaagtg tgcccagctg 840
gctggaaacc tggcagtgat accatcaagc ctgatgtcca aaagagcaaa gaatatttct 900
ccaagcagaa gtgagcgctg ggctgtttta gtgccaggct gcggtgggca gccatgagaa 960
caaaacctct tctgtatttt tttttccat tagtaaaaca caagacttca gattcagccg 1020
aattgtggtg tcttacaagg caggcctttc ctacaggggg tggagagacc agcctttctt 1080
cctttggtag gaatggcctg agttggcgtt gtgggcaggc tactggtttg tatgatgtat 1140
tagtagagca acccattaat cttttgtagt ttgtattaaa cttgaactga gaccttgatg 1200
agtctttaaa aaaaaaaaaa aaaaaaaaaa aaaaaa 1236

<210> 29
<211> 1042
<212> DNA
<213> Homo sapiens

<400> 29

```
actctcgcga gatccctact ggctataaag gcagcgcccc ggagagctct tgcgcgtctt      60
gttcttgcct ggtgtcggtg gttagtttct gcgacttgtg ttgggactgc tgataggaag     120
atgtcttcag gaaatgctaa aattgggcac cctgccccca acttcaaagc cacagctgtt     180
atgccagatg gtcagtttaa agatatcagc ctgtctgact acaaaggaaa atatgttgtg     240
ttcttctttt accctcttga cttcaccttt gtgtgcccca cggagatcat tgctttcagt     300
gatagggcag aagaatttaa gaaactcaac tgccaagtga ttggtgcttc tgtggattct     360
cacttctgtc atctagcatg ggtcaataca cctaagaaac aaggaggact gggacccatg     420
aacattcctt tggtatcaga cccgaagcgc accattgctc aggattatgg ggtcttaaag     480
gctgatgaag gcatctcgtt caggggcctt tttatcattg atgataaggg tattcttcgg     540
cagatcactg taaatgacct ccctgttggc cgctctgtgg atgagacttt gagactagtt     600
caggccttcc agttcactga caaacatggg gaagtgtgcc cagctggctg gaaacctggc     660
agtgatacca tcaagcctga tgtccaaaag agcaaagaat atttctccaa gcagaagtga     720
gcgctgggct gttttagtgc caggctgcgg tgggcagcca tgagaacaaa acctcttctg     780
tatttttttt ttccattagt aaaacacaag acttcagatt cagccgaatt gtggtgtctt     840
acaaggcagg cctttcctac agggggtgga gagaccagcc tttcttcctt tggtaggaat     900
ggcctgagtt ggcgttgtgg gcaggctact ggtttgtatg atgtattagt agagcaaccc     960
attaatcttt tgtagtttgt attaaacttg aactgagacc ttgatgagtc tttaaaaaaa    1020
aaaaaaaaaa aaaaaaaaaa aa                                             1042
```

<210> 30
<211> 1035
<212> DNA
<213> Homo sapiens

<400> 30

gcggtgccct tgcggcgcag ctggggtcgc ggccctgctc cccgcgcttt cttaaggccc 60
gcgggcggcg caggagcggc actcgtggct gtggtggctt cggcagcggc ttcagcagat 120
cggcggcatc agcggtagca ccagcactag cagcatgttg agccgggcag tgtgcggcac 180
cagcaggcag ctggctccgg ttttggggta tctgggctcc aggcagaagc acagcctccc 240
cgacctgccc tacgactacg gcgccctgga acctcacatc aacgcgcaga tcatgcagct 300
gcaccacagc aagcaccacg cggcctacgt gaacaacctg aacgtcaccg aggagaagta 360
ccaggaggcg ttggccaagg gagatgttac agcccagata gctcttcagc ctgcactgaa 420
gttcaatggt ggtggtcata tcaatcatag cattttctgg acaaacctca gccctaacgg 480
tggtggagaa cccaaagggg agttgctgga agccatcaaa cgtgactttg gttcctttga 540
caagtttaag gagaagctga cggctgcatc tgttggtgtc caaggctcag gttggggttg 600
gcttggtttc aataaggaac ggggacactt acaaattgct gcttgtccaa atcaggatcc 660
actgcaagga acaacaggcc ttattccact gctggggatt gatgtgtggg agcacgctta 720
ctaccttcag tataaaaatg tcaggcctga ttatctaaaa gctatttgga atgtaatcaa 780
ctgggagaat gtaactgaaa gatacatggc ttgcaaaaag taaaccacga tcgttatgct 840
gatcataccc taatgatccc agcaagataa tgtcctgtct tctaagatgt gcatcaagcc 900
tggtacatac tgaaaaccct ataaggtcct ggataatttt tgtttgatta ttcattgaag 960
aaacatttat tttccaattg tgtgaagttt ttgactgtta ataaaagaat ctgtcaacca 1020
tcaaaaaaaa aaaaa 1035

<210> 31
<211> 918
<212> DNA
<213> Homo sapiens

<400> 31

gcggtgccct tgcggcgcag ctggggtcgc ggccctgctc cccgcgcttt cttaaggccc 60
gcgggcggcg caggagcggc actcgtggct gtggtggctt cggcagcggc ttcagcagat 120
cggcggcatc agcggtagca ccagcactag cagcatgttg agccgggcag tgtgcggcac 180

```
cagcaggcag ctggctccgg ttttggggta tctgggctcc aggcagaagc acagcctccc       240
cgacctgccc tacgactacg gcgccctgga acctcacatc aacgcgcaga tcatgcagct       300
gcaccacagc aagcaccacg cggcctacgt gaacaacctg aacgtcaccg aggagaagta       360
ccaggaggcg ttggccaagg gggagttgct ggaagccatc aaacgtgact ttggttcctt       420
tgacaagttt aaggagaagc tgacggctgc atctgttggt gtccaaggct caggttgggg       480
ttggcttggt ttcaataagg aacggggaca cttacaaatt gctgcttgtc caaatcagga       540
tccactgcaa ggaacaacag gccttattcc actgctgggg attgatgtgt gggagcacgc       600
ttactacctt cagtataaaa atgtcaggcc tgattatcta aaagctattt ggaatgtaat       660
caactgggag aatgtaactg aaagatacat ggcttgcaaa aagtaaacca cgatcgttat       720
gctgatcata ccctaatgat cccagcaaga taatgtcctg tcttctaaga tgtgcatcaa       780
gcctggtaca tactgaaaac cctataaggt cctggataat tttgtttga ttattcattg       840
aagaaacatt tattttccaa ttgtgtgaag tttttgactg ttaataaaag aatctgtcaa       900
ccatcaaaaa aaaaaaaa                                                      918
```

<210> 32
<211> 18
<212> DNA
<213> Artificial sequence

<220>
<223> sense oligonucleotide for CAT

<400> 32
cgcagttcgg ttctccac 18

<210> 33
<211> 18
<212> DNA
<213> Artificial sequence

<220>
<223> antisense oligonucleotide for CAT

<400> 33
gggtcccgaa ctgtgtca 18

<210> 34
<211> 20
<212> DNA
<213> Artificial sequence

<220>
<223> sense oligonucleotide for SOD1

<400> 34
gcatcatcaa tttcgagcag 20

<210> 35
<211> 20
<212> DNA
<213> Artificial sequence

<220>
<223> antisense oligonucleotide for SOD1

<400> 35
caggccttca gtcagtcctt 20

<210> 36
<211> 19
<212> DNA
<213> Artificial sequence

<220>
<223> sense oligonucleotide for GPX1

<400> 36
caaccagttt gggcatcag 19

<210> 37
<211> 19
<212> DNA
<213> Artificial sequence

<220>
<223> antisense oligonucleotide for GPX1

<400> 37
gttcacctcg cacttctcg 19

<210> 38
<211> 18
<212> DNA
<213> Artificial sequence

<220>
<223> sense oligonucleotide for GPX4

<400> 38
tacggaccca tggaggag 18

<210> 39
<211> 22
<212> DNA
<213> Artificial sequence

<220>
<223> antisense oligonucleotide for GPX4

<400> 39
ccacacactt gtggagctag aa 22

<210> 40
<211> 21
<212> DNA

<213> Artificial sequence

<220>
<223> sense oligonucleotide for PRDX2

<400> 40
cactgacaaa catggggaag t 21

<210> 41
<211> 21
<212> DNA
<213> Artificial sequence

<220>
<223> antisense oligonucleotide for PRDX2

<400> 41
tttgctcttt tggacatcag g 21

<210> 42
<211> 20
<212> DNA
<213> Artificial sequence

<220>
<223> sense oligonucleotide for SO2

<400> 42
tccactgcaa ggaacaacag 20

<210> 43
<211> 19
<212> DNA
<213> Artificial sequence

<220>
<223> antisense oligonucleotide for SOD2

<400> 43
taagcgtgct cccacacat 19

<210> 44
<211> 18
<212> DNA
<213> Artificial sequence

<220>
<223> sense oligonucleotide for GPX2

<400> 44
gtccttggct tcccttgc 18

<210> 45
<211> 22
<212> DNA
<213> Artificial sequence

<220>

<223> antisense oligonucleotide for GPX2

<400> 45
tgttcaggat ctcctcattc tg 22

<210> 46
<211> 19
<212> DNA
<213> Artificial sequence

<220>
<223> sense oligonucleotide for GAPDH

<400> 46
agccacatcg ctcagacac 19

<210> 47
<211> 19
<212> DNA
<213> Artificial sequence

<220>
<223> antisense oligonucleotide for GAPDH

<400> 47
gcccaatacg accaaatcc 19